# EUROPEAN PATENT APPLICATION

(11) **EP 4 375 298 A1**
(43) Date of publication of application: **29.05.2024**
(21) Application number: 22845392.4
(22) Date of filing: 21.07.2022
(51) Int. Cl.: C07K 16/22, A61K 39/395

(54) **PHARMACEUTICAL COMPOSITION OF ANTI-ANGPTL3 ANTIBODY OR ANTIGEN BINDING FRAGMENT THEREOF AND ITS APPLICATION**

(30) Priority: 21.07.2021 CN 202110822526
(71) Applicant: Jiangsu Hengrui Pharmaceuticals Co., Ltd., Lianyungang, Jiangsu 222047 (CN); Shanghai Hengrui Pharmaceutical Co., Ltd., Shanghai 200245 (CN)
(72) Inventor: YANG, Xiqin, Lianyungang, Jiangsu 222047 (CN); GE, Lingxiao, Lianyungang, Jiangsu 222047 (CN); WANG, Hongwei, Lianyungang, Jiangsu 222047 (CN)
(74) Representative: Regimbeau
(86) International application number: PCT/CN2022/106993
(87) International publication number: WO 2023/001228

(57) **Abstract**

A pharmaceutical composition of an anti-ANGPTL3 antibody or an antigen binding fragment thereof and its application. Specifically, the pharmaceutical composition comprises an anti-ANGPTL3 antibody or an antigen binding fragment thereof and an acetic acid sodium acetate buffer. The pharmaceutical composition may also comprise a sugar and a surfactant. The pharmaceutical composition has good antibody stability.

## Description

The present application claims priority to Chinese Patent Application No. 202110822526.8 filed on July 21, 2021.

### TECHNICAL FIELD

The present disclosure relates to the field of pharmaceutical formulations and in particular to a pharmaceutical composition comprising an anti-ANGPTL3 antibody or an antigen-binding fragment thereof, and pharmaceutical use thereof.

### BACKGROUND

Angiopoietin-like protein 3 (ANGPTL3) is a secreted protein, and as a member of the angiopoietin-like protein family, it is mainly expressed in the liver. The amino-terminal coil region of ANGPTL3 is associated with the inhibition of lipoprotein lipase (LPL) activity, and the modulation of the carboxy-terminal fibrinogen-like regions of triglycerides (TG) is primarily associated with the modulation of angiogenesis. Thus, the protein has the function of modulating lipid metabolism and promoting angiogenesis. ANGPTL3 serves primarily to modulate lipoprotein metabolism and, by inhibition of LPL and endothelial lipase (EL), plays a role in modulating lipid metabolism. At present, ANGPTL3 inhibitors fall into 2 categories, one of which is ANGPTL3-ASO, and the other is ANGPTL3 monoclonal antibodies such as evinacumab. Their mechanisms and sites of action are different-ANGPTL3-ASO mainly acts on hepatocytes, while evinacumab takes effect in blood circulation. However, inhibition of ANGPTL3, whether at the phenotypic or genetic level, has a remarkable and stable lipid lowering effect. Monoclonal antibodies of the IgG4 subtype that target ANGPTL3 can bind to ANGPTL3 and, by blocking the binding of ANGPTL3 to LPL, remove the ANGPTL3's inhibition of LPL enzyme activity and promote triglyceride degradation in blood plasma, thereby fulfilling the purpose of treating hyperlipidemia.

WO2021147984A provides an anti-ANGPTL3 antibody and an antigen-binding fragment thereof with a better and more lasting lipid lowering effect. Due to their great molecular weights and complex structures, antibody drugs are prone to degradation, aggregation, unwanted chemical modifications, etc., which make them unstable. It is particularly important to develop stable formulations of antibody drugs that make the antibodies more suitable for administration, keep the antibodies stable during storage and subsequent use, and make the antibodies produce better therapeutic effects. Thus, there is a need to develop relatively stable formulations of anti-ANGPTL3 antibodies and antigen-binding fragments thereof. The present disclosure provides a pharmaceutical composition comprising an anti-ANGPTL3 antibody and an antigen-binding fragment thereof that is sufficiently stable, has good lyophilization morphology, and is more suitable for administration.

### SUMMARY

The present disclosure provides a pharmaceutical composition, comprising an anti-ANGPTL3 antibody or an antigen-binding fragment thereof, and a buffer. Also, the present disclosure provides a method for treating and preventing disease using the pharmaceutical composition, and pharmaceutical use thereof.

In some embodiments, the aforementioned buffer is selected from any of an acetate buffer, a histidine buffer, a phosphate buffer, a succinate buffer and a citric acid buffer, for example, an acetate buffer.

In some embodiments, the aforementioned buffer is selected from the group consisting of any of acetic acid-sodium acetate, histidine-hydrochloride, sodium dihydrogen phosphate-disodium hydrogen phosphate, succinic acid-sodium succinate, and citric acid-sodium citrate buffers, for example, an acetic acid-sodium acetate buffer.

In some embodiments, the anti-ANGPTL3 antibody or the antigen-binding fragment thereof in the pharmaceutical composition comprises an antibody heavy chain variable region and an antibody light chain variable region, wherein: the heavy chain variable region comprises the HCDR1, HCDR2 and HCDR3 of a heavy chain variable region of the amino acid sequence set forth in SEQ ID NO: 9, and/or the light chain variable region comprises the LCDR1, LCDR2 and LCDR3 of a light chain variable region of the amino acid sequence set forth in SEQ ID NO: 10. The CDRs are defined according to the Kabat, IMGT, Chothia, AbM or Contact numbering scheme. In some specific embodiments, the CDRs are defined according to the Kabat numbering scheme.

In some embodiments, the anti-ANGPTL3 antibody or the antigen-binding fragment thereof in the pharmaceutical composition comprises an antibody heavy chain variable region and an antibody light chain variable region, wherein: the heavy chain variable region comprises a HCDR1, the amino acid sequence of which is set forth in SEQ ID NO: 14; a HCDR2, the amino acid sequence of which is LINPRDDSTSYAQKFQG (SEQ ID NO: 23); and a HCDR3, the amino acid sequence of which is set forth in SEQ ID NO: 16; and/or the light chain variable region comprises a LCDR1, the amino acid sequence of which is RSSQSLLHSNGYTYLD (SEQ ID NO: 24); a LCDR2, the amino acid sequence of which is set forth in SEQ ID NO: 12; and a LCDR3, the amino acid sequence of which is set forth in SEQ ID NO: 13.

In some embodiments, the anti-ANGPTL3 antibody or the antigen-binding fragment thereof in the pharmaceutical composition comprises an antibody heavy chain variable region and an antibody light chain variable region, wherein: the heavy chain variable region comprises the HCDR1, HCDR2 and HCDR3 of a heavy chain variable region of the amino acid sequence set forth in SEQ ID NO: 17, and/or the light chain variable region comprises the LCDR1, LCDR2 and LCDR3 of a light chain variable region of the amino acid sequence set forth in SEQ ID NO: 18. The CDRs are defined according to the Kabat, IMGT, Chothia, AbM or Contact numbering scheme. In some specific embodiments, the CDRs are defined according to the Kabat numbering scheme.

In some embodiments, the anti-ANGPTL3 antibody or the antigen-binding fragment thereof in the pharmaceutical composition comprises an antibody heavy chain variable region and an antibody light chain variable region, wherein: the heavy chain variable region comprises a HCDR1, a HCDR2 and a HCDR3, the amino acid sequences of which are set forth in SEQ ID NO: 14, SEQ ID NO: 15 and SEQ ID NO: 16, respectively; and/or the light chain variable region comprises a LCDR1, a LCDR2 and a LCDR3, the amino acid sequences of which are set forth in SEQ ID NO: 11, SEQ ID NO: 12 and SEQ ID NO: 13, respectively.

In some embodiments, the anti-ANGPTL3 antibody or the antigen-binding fragment thereof in the pharmaceutical composition is selected from the group consisting of a murine antibody, a chimeric antibody, a humanized antibody, and a fully human antibody; in some specific embodiments, the anti-ANGPTL3 antibody or the antigen-binding fragment thereof in the pharmaceutical composition is a humanized antibody.

In some embodiments, the light and heavy chain FR sequences in the light and heavy chain variable regions of the humanized ANGPTL3 antibody in the pharmaceutical composition are derived from human germline light and heavy chain FRs or mutated sequences thereof, respectively.

In some embodiments, the anti-ANGPTL3 antibody or the antigen-binding fragment thereof in the pharmaceutical composition comprises an antibody heavy chain variable region and an antibody light chain variable region, wherein: the amino acid sequence of the heavy chain variable region is set forth in SEQ ID NO: 9 or has at least 80%, at least 85%, at least 90% or more sequence identity thereto, and/or the amino acid sequence of the light chain variable region is set forth in SEQ ID NO: 10 or has at least 80%, at least 85%, at least 90% or more sequence identity thereto.

In some embodiments, the anti-ANGPTL3 antibody or the antigen-binding fragment thereof in the pharmaceutical composition comprises an antibody heavy chain variable region and an antibody light chain variable region, wherein: the amino acid sequence of the heavy chain variable region is set forth in SEQ ID NO: 17 or has at least 80%, at least 85%, at least 90% and more sequence identity thereto, and/or the amino acid sequence of the light chain variable region is set forth in SEQ ID NO: 18 or has at least 80%, at least 85%, at least 90% and more sequence identity thereto.

In the present disclosure, "at least 90% and more sequence identity" encompasses at least 90%, at least 91%, at least 92%, at least 93%, at least 94%, at least 95%, at least 96%, at least 97%, at least 98%, at least 99% and more sequence identity.

In some embodiments, the anti-ANGPTL3 antibody or the antigen-binding fragment thereof in the pharmaceutical composition comprises an antibody heavy chain variable region and an antibody light chain variable region, wherein: the amino acid sequence of the heavy chain variable region is set forth in SEQ ID NO: 9, and the amino acid sequence of the light chain variable region is set forth in SEQ ID NO: 10.

In some embodiments, the anti-ANGPTL3 antibody or the antigen-binding fragment thereof in the pharmaceutical composition comprises an antibody heavy chain variable region and an antibody light chain variable region, wherein: the amino acid sequence of the heavy chain variable region is set forth in SEQ ID NO: 17, and the amino acid sequence of the light chain variable region is set forth in SEQ ID NO: 18.

In some embodiments, the anti-ANGPTL3 antibody or the antigen-binding fragment thereof further comprises a heavy chain constant region and a light chain constant region. In alternative embodiments, the heavy chain constant region is derived from human IgG1, IgG2, IgG3 and IgG4 constant regions, and the light chain constant region is derived from human κ and λ chain constant regions.

In some embodiments, the amino acid sequence of the heavy chain constant region is set forth in SEQ ID NO: 19 or has at least 80%, at least 85%, at least 90% and more sequence identity thereto, and/or the amino acid sequence of the light chain constant region is set forth in SEQ ID NO: 20 or has at least 80%, at least 85%, at least 90% and more sequence identity thereto.

In some embodiments, the anti-ANGPTL3 antibody or the antigen-binding fragment thereof comprises a heavy chain and a light chain, wherein: the amino acid sequence of the heavy chain is set forth in SEQ ID NO: 21 or has at least 80%, at least 85%, at least 90% and more sequence identity thereto, and/or the amino acid sequence of the light chain is set forth in SEQ ID NO: 22 or has at least 80%, at least 85%, at least 90% and more sequence identity thereto.

In some embodiments, the anti-ANGPTL3 antibody or the antigen-binding fragment thereof in the pharmaceutical composition comprises an antibody heavy chain and an antibody light chain, wherein: the amino acid sequence of the heavy chain is set forth in SEQ ID NO: 21, and the amino acid sequence of the light chain is set forth in SEQ ID NO: 22.

In some embodiments, the antigen-binding fragment described in the present disclosure is selected from the group consisting of: Fab, F(ab')2, Fab', Fd, Fv, dsFv, scFv, Fab and bispecific antibodies.

In some embodiments, the buffer in the pharmaceutical composition has a pH of 4.0 to 7.0, for example, 4.0 to 6.9, 4.0 to 6.8, 4.0 to 6.7, 4.0 to 6.6, 4.0 to 6.5, 4.0 to 6.4, 4.0 to 6.3, 4.0 to 6.2, 4.0 to 6.1, 4.0 to 6.0, 4.0 to 5.9, 4.0 to 5.8, 4.0 to 5.7, 4.0 to 5.6, 4.0 to 5.5, 4.5 to 6.5, 4.5 to 6.0, 4.5 to 5.5, 5.0 to 7.0, 5.0 to 6.5, 5.0 to 6.0, 5.0 to 5.5, 4.6 to 6.6, 4.6 to 6.5, 4.6 to 6.4, 4.6 to 6.3, 4.6 to 6.2, 4.6 to 6.1, 4.6 to 6.0, 4.6 to 5.9, 4.6 to 5.8, 4.6 to 5.7, 4.6 to 5.6, 4.6 to 5.5, 4.6 to 5.4, 4.8 to 6.6, 4.8 to 6.5, 4.8 to 6.4, 4.8 to 6.3, 4.8 to 6.2, 4.8 to 6.1, 4.8 to 6.0, 4.8 to 5.9, 4.8 to 5.8, 4.8 to 5.7, 4.8 to 5.6, 4.8 to 5.5, 4.8 to 5.4, 4.9 to 5.4, or 5.0 to 5.4; in some specific embodiments, the buffer has a pH of about 4.0, about 4.1, about 4.2, about 4.3, about 4.4, about 4.5, about 4.6, about 4.7, about 4.8, about 4.9, about 5.0, about 5.1, about 5.2, about 5.3, about 5.4, about 5.5, about 5.6, about 5.7, about 5.8, about 5.9, about 6.0, about 6.5, about 6.6, about 6.7, about 6.8, about 6.9, or about 7.0, for example, about 5.2.

In some embodiments, the concentration of the buffer in the pharmaceutical composition is 5 mM to 40 mM, for example, 5 mM to 30 mM, 5 mM to 20 mM, 5 mM to 15 mM, 5 mM to 10 mM, 10 mM to 15 mM, or 8 mM to 12 mM; in some specific embodiments, the concentration of the buffer is about 5 mM, about 6 mM, about 7 mM, about 8 mM, about 9 mM, about 10 mM, about 11 mM, about 12 mM, about 13 mM, about 14 mM, about 15 mM, about 18 mM, or about 20 mM, for example, about 10 mM.

In some embodiments, the concentration of the anti-ANGPTL3 antibody or the antigen-binding fragment thereof in the pharmaceutical composition is 10 mg/mL to 200 mg/mL, for example, 50 mg/mL to 200 mg/mL, 50 mg/mL to 150 mg/mL, 60 mg/mL to 150 mg/mL, 60 mg/mL to 120 mg/mL, 80 mg/mL to 150 mg/mL, 80 mg/mL to 120 mg/mL, 90 mg/mL to 120 mg/mL, or 90 mg/mL to 110 mg/mL; in some specific embodiments, the concentration of the anti-ANGPTL3 antibody or the antigen-binding fragment thereof is about 10 mg/mL, about 20 mg/mL, about 30 mg/mL, about 50 mg/mL, about 60 mg/mL, about 65 mg/mL, about 70 mg/mL, about 75 mg/mL, about 80 mg/mL, about 85 mg/mL, about 90 mg/mL, about 95 mg/mL, about 100 mg/mL, about 105 mg/mL, about 110 mg/mL, about 115 mg/mL, about 120 mg/mL, about 130 mg/mL, about 140 mg/mL, about 150 mg/mL, about 160 mg/mL, about 170 mg/mL, about 180 mg/mL, about 190 mg/mL, about 200 mg/mL, or any value between any two of these values, for example, about 100 mg/mL.

In some embodiments, the surfactant in the pharmaceutical composition may be selected from the group consisting of polysorbate 20, polysorbate 80, poloxamer, Triton, sodium dodecyl sulfonate, sodium lauryl sulfonate, sodium octyl glycoside, lauryl-sulfobetaine, myristyl-sulfobetaine, linoleyl-sulfobetaine, stearyl-sulfobetaine, lauryl-sarcosine, myristyl-sarcosine, linoleyl-sarcosine, stearyl-sarcosine, linoleyl-betaine, myristyl-betaine, cetyl-betaine, lauramido propyl-betaine, cocaramide propyl-betaine, linoleinamide propyl-betaine, myristylamide propyl-betaine, palmitamide propyl-betaine, isostearamide propyl-betaine, myristylamide propyl-dimethylamine, palmitamide propyl-dimethylamine, isostearamide propyl-dimethylamine, sodium methyl cocoyl, sodium methyl oleyl taurate, polyethylene glycol, polypropylene glycol, copolymer of ethylene and propylene glycol, and the like. In some specific embodiments, the surfactant is polysorbate 80 or polysorbate 20, for example, polysorbate 80.

In some embodiments, the concentration of the surfactant in the pharmaceutical composition is 0.05 mg/mL to 0.6 mg/mL, for example, 0.1 mg/mL to 0.6 mg/mL, 0.1 mg/mL to 0.5 mg/mL, or 0.1 mg/mL to 0.4 mg/mL, for example, 0.1 mg/mL to 0.3 mg/mL; in some specific embodiments, the concentration of the surfactant in the pharmaceutical composition is about 0.1 mg/mL, about 0.15 mg/mL, about 0.2 mg/mL, about 0.25 mg/mL, about 0.3 mg/mL, about 0.4 mg/mL, about 0.5 mg/mL, or about 0.6 mg/mL, for example, about 0.2 mg/mL.

In some embodiments, the pharmaceutical composition further comprises a stabilizer. In alternative embodiments, the stabilizer is selected from the group consisting of a sugar and an amino acid. In alternative embodiments, the sugar includes the general composition (CH₂O)ₙ and derivatives thereof, including monosaccharides, disaccharides, trisaccharides, polysaccharides, sugar alcohols, reducing sugars, non-reducing sugars, and the like. In alternative embodiments, the sugar may be selected from the group consisting of glucose, sucrose, trehalose, lactose, fructose, maltose, dextran, glycerin, erythritol, glycerol, arabitol, xylose, sorbitol, mannitol, mellibiose, melezitose, raffinose, mannotriose, stachyose, maltose, lactulose, maltulose, glucitol, maltitol, lactitol, iso-maltulose, and the like. In some specific embodiments, the sugar is a non-reducing disaccharide, such as sucrose. In some specific embodiments, the amino acid is selected from the group consisting of glycine, methionine and proline.

In some embodiments, the concentration of the sugar in the pharmaceutical composition is 40 mg/mL to 110 mg/mL, for example, 40 mg/mL to 95 mg/mL, 60 mg/mL to 90 mg/mL, 60 mg/mL to 80 mg/mL, 70 mg/mL to 90 mg/mL, or 70 mg/mL to 80 mg/mL; in some specific embodiments, the concentration of the sugar is about 60 mg/mL, about 65 mg/mL, about 70 mg/mL, about 75 mg/mL, about 80 mg/mL, about 85 mg/mL, about 90 mg/mL, or any value between any two of these values, for example, about 75 mg/mL.

The present disclosure provides a pharmaceutical composition, comprising:
(a) 10 mg/mL to 200 mg/mL (e.g., about 50 mg/mL) anti-ANGPTL3 antibody or antigen-binding fragment thereof, and
(b) about 10 mM histidine-histidine hydrochloride buffer;
optionally, the pharmaceutical composition has a pH of 6.0 to 6.6 (e.g., about 6.0 or about 6.6).

The present disclosure provides a pharmaceutical composition, comprising:
(a) 10 mg/mL to 200 mg/mL (e.g., about 50 mg/mL) anti-ANGPTL3 antibody or antigen-binding fragment thereof, and
(b) about 10 mM acetic acid-sodium acetate buffer;
optionally, the pharmaceutical composition has a pH of 4.6 to 5.0 (e.g., about 4.6 or about 5.0).

The present disclosure provides a pharmaceutical composition, comprising:
(a) 10 mg/mL to 200 mg/mL anti-ANGPTL3 antibody or antigen-binding fragment thereof, and
(b) 5 mM to 30 mM acetate buffer (e.g., acetic acid-sodium acetate buffer).

In some embodiments, the pharmaceutical composition comprises:
(a) 10 mg/mL to 200 mg/mL anti-ANGPTL3 antibody or antigen-binding fragment thereof,
(b) 5 mM to 30 mM acetate buffer (e.g., acetic acid-sodium acetate buffer),
(c) 40 mg/mL to 90 mg/mL sugar (e.g., sucrose), and
(d) 0.05 mg/mL to 0.6 mg/mL surfactant (e.g., polysorbate 80 or polysorbate 20);
optionally, the pharmaceutical composition has a pH of 4.5 to 6.5, for example, 5.0 to 5.4, for example about 5.2.

The present disclosure provides a pharmaceutical composition, comprising:
(a) 10 mg/mL to 200 mg/mL anti-ANGPTL3 antibody or antigen-binding fragment thereof, and
(b) 5 mM to 20 mM acetate buffer (e.g., acetic acid-sodium acetate buffer);
optionally, the pharmaceutical composition has a pH of 5.0 to 5.5.

In some embodiments, the pharmaceutical composition comprises:
(a) 50 mg/mL to 150 mg/mL anti-ANGPTL3 antibody or antigen-binding fragment thereof,
(b) 5 mM to 15 mM acetate buffer (e.g., acetic acid-sodium acetate buffer),
(c) 60 mg/mL to 90 mg/mL sucrose, and
(d) 0.05 mg/mL to 0.5 mg/mL polysorbate 80;
optionally, the pharmaceutical composition has a pH of 5.0 to 6.0.

In some embodiments, the pharmaceutical composition comprises:
(a) 40 mg/mL to 120 mg/mL anti-ANGPTL3 antibody or antigen-binding fragment thereof,
(b) 5 mM to 15 mM acetate buffer (e.g., acetic acid-sodium acetate buffer),
(c) 60 mg/mL to 90 mg/mL sucrose, and
(d) 0.1 mg/mL to 0.4 mg/mL polysorbate 80;
optionally, the pharmaceutical composition has a pH of 5.0 to 5.8.

In some embodiments, the pharmaceutical composition comprises:
(a) 80 mg/mL to 120 mg/mL anti-ANGPTL3 antibody or antigen-binding fragment thereof,
(b) 5 mM to 15 mM acetate buffer (e.g., acetic acid-sodium acetate buffer),
(c) 70 mg/mL to 90 mg/mL sucrose, and
(d) 0.1 mg/mL to 0.3 mg/mL polysorbate 80;
optionally, the pharmaceutical composition has a pH of 5.0 to 5.5.

In some embodiments, the pharmaceutical composition comprises:
(a) 80 mg/mL to 120 mg/mL anti-ANGPTL3 antibody or antigen-binding fragment thereof,
(b) 5 mM to 15 mM acetate buffer (e.g., acetic acid-sodium acetate buffer),
(c) 70 mg/mL to 90 mg/mL sucrose, and
(d) 0.1 mg/mL to 0.3 mg/mL polysorbate 80;
optionally, the pharmaceutical composition has a pH of 5.0 to 5.5.

In some embodiments, the pharmaceutical composition comprises:
(a) 90 mg/mL to 110 mg/mL anti-ANGPTL3 antibody or antigen-binding fragment thereof,
(b) 8 mM to 12 mM acetate buffer (e.g., acetic acid-sodium acetate buffer),
(c) 75 mg/mL to 85 mg/mL sucrose, and
(d) 0.15 mg/mL to 0.25 mg/mL polysorbate 80;
optionally, the pharmaceutical composition has a pH of 5.1 to 5.3.

In some embodiments, the pharmaceutical composition comprises:
(a) about 100 mg/mL anti-ANGPTL3 antibody or antigen-binding fragment thereof,
(b) 5 mM to 15 mM acetic acid-sodium acetate buffer,
(c) 60 mg/mL to 90 mg/mL sucrose, and
(d) 0.1 mg/mL to 0.4 mg/mL polysorbate 80;
optionally, the pharmaceutical composition has a pH of 5.0 to 5.4.

In some specific embodiments, the pharmaceutical composition comprises:
(1) about 100 mg/mL anti-ANGPTL3 antibody or antigen-binding fragment thereof, about 75 mg/mL sucrose, about 0.1 mg/mL polysorbate 80, and about 10 mM acetic acid-sodium acetate buffer, the pH being 5.0 to 5.4 (e.g., about 5.0, about 5.1, about 5.2, about 5.3, or about 5.4);
(2) about 100 mg/mL anti-ANGPTL3 antibody or antigen-binding fragment thereof, about 75 mg/mL sucrose, about 0.2 mg/mL polysorbate 80, and about 10 mM acetic acid-sodium acetate buffer, the pH being 5.0 to 5.4 (e.g., about 5.0, about 5.1, about 5.2, about 5.3, or about 5.4);
(3) about 100 mg/mL anti-ANGPTL3 antibody or antigen-binding fragment thereof, about 75 mg/mL sucrose, about 0.4 mg/mL polysorbate 80, and about 10 mM acetic acid-sodium acetate buffer, the pH being 5.0 to 5.4 (e.g., about 5.0, about 5.1, about 5.2, about 5.3, or about 5.4);
(4) about 80 mg/mL anti-ANGPTL3 antibody or antigen-binding fragment thereof, about 75 mg/mL sucrose, about 0.2 mg/mL polysorbate 80, and about 10 mM acetic acid-sodium acetate buffer, the pH being 5.0 to 5.4 (e.g., about 5.0, about 5.1, about 5.2, about 5.3, or about 5.4);
(5) about 120 mg/mL anti-ANGPTL3 antibody or antigen-binding fragment thereof, about 75 mg/mL sucrose, about 0.2 mg/mL polysorbate 80, and about 10 mM acetic acid-sodium acetate buffer, the pH being 5.0 to 5.4 (e.g., about 5.0, about 5.1, about 5.2, about 5.3, or about 5.4);
(6) about 150 mg/mL anti-ANGPTL3 antibody or antigen-binding fragment thereof, about 75 mg/mL sucrose, about 0.2 mg/mL polysorbate 80, and about 10 mM acetic acid-sodium acetate buffer, the pH being 5.0 to 5.4 (e.g., about 5.0, about 5.1, about 5.2, about 5.3, or about 5.4);
(7) about 100 mg/mL anti-ANGPTL3 antibody or antigen-binding fragment thereof, about 60 mg/mL sucrose, about 0.2 mg/mL polysorbate 80, and about 10 mM acetic acid-sodium acetate buffer, the pH being 5.0 to 5.4 (e.g., about 5.0, about 5.1, about 5.2, about 5.3, or about 5.4);
(8) about 100 mg/mL anti-ANGPTL3 antibody or antigen-binding fragment thereof, about 65 mg/mL sucrose, about 0.2 mg/mL polysorbate 80, and about 10 mM acetic acid-sodium acetate buffer, the pH being 5.0 to 5.4 (e.g., about 5.0, about 5.1, about 5.2, about 5.3, or about 5.4);
(9) about 100 mg/mL anti-ANGPTL3 antibody or antigen-binding fragment thereof, about 70 mg/mL sucrose, about 0.2 mg/mL polysorbate 80, and about 10 mM acetic acid-sodium acetate buffer, the pH being 5.0 to 5.4 (e.g., about 5.0, about 5.1, about 5.2, about 5.3, or about 5.4);
(10) about 100 mg/mL anti-ANGPTL3 antibody or antigen-binding fragment thereof, about 80 mg/mL sucrose, about 0.2 mg/mL polysorbate 80, and about 10 mM acetic acid-sodium acetate buffer, the pH being 5.0 to 5.4 (e.g., about 5.0, about 5.1, about 5.2, about 5.3, or about 5.4);
(11) about 100 mg/mL anti-ANGPTL3 antibody or antigen-binding fragment thereof, about 90 mg/mL sucrose, about 0.2 mg/mL polysorbate 80, and about 10 mM acetic acid-sodium acetate buffer, the pH being 5.0 to 5.4 (e.g., about 5.0, about 5.1, about 5.2, about 5.3, or about 5.4);
(12) about 100 mg/mL anti-ANGPTL3 antibody or antigen-binding fragment thereof, about 75 mg/mL sucrose, about 0.2 mg/mL polysorbate 80, and about 5 mM acetic acid-sodium acetate buffer, the pH being 5.0 to 5.4 (e.g., about 5.0, about 5.1, about 5.2, about 5.3, or about 5.4); or
(13) about 100 mg/mL anti-ANGPTL3 antibody or antigen-binding fragment thereof, about 75 mg/mL sucrose, about 0.2 mg/mL polysorbate 80, and about 15 mM acetic acid-sodium acetate buffer, the pH being 5.0 to 5.4 (e.g., about 5.0, about 5.1, about 5.2, about 5.3, or about 5.4).

In the above embodiments, the anti-ANGPTL3 antibody or the antigen-binding fragment thereof is selected from the group consisting of:
1) an anti-ANGPTL3 antibody or an antigen-binding fragment thereof, comprising a heavy chain variable region and a light chain variable region, wherein the light chain variable region comprises a LCDR1, a LCDR2 and a LCDR3, the amino acid sequences of which are set forth in SEQ ID NO: 11, SEQ ID NO: 12 and SEQ ID NO: 13, respectively; and the heavy chain variable region comprises a HCDR1, a HCDR2 and a HCDR3, the amino acid sequences of which are set forth in SEQ ID NO: 14, SEQ ID NO: 15 and SEQ ID NO:16, respectively;
2) an anti-ANGPTL3 antibody or an antigen-binding fragment thereof, comprising an antibody heavy chain variable region and an antibody light chain variable region, wherein the amino acid sequence of the heavy chain variable region is set forth in SEQ ID NO: 17, and the amino acid sequence of the light chain variable region is set forth in SEQ ID NO: 18;
3) an anti-ANGPTL3 antibody or an antigen-binding fragment thereof, comprising an antibody heavy chain and an antibody light chain, wherein the amino acid sequence of the heavy chain is set forth in SEQ ID NO: 21, and the amino acid sequence of the light chain is set forth in SEQ ID NO: 22;
4) an anti-ANGPTL3 antibody or an antigen-binding fragment thereof, comprising a heavy chain variable region and a light chain variable region, wherein the light chain variable region comprises a LCDR1, a LCDR2 and a LCDR3, the amino acid sequences of which are set forth in SEQ ID NO:24, SEQ ID NO: 12 and SEQ ID NO: 13, respectively; and the heavy chain variable region comprises a HCDR1, a HCDR2 and a HCDR3, the amino acid sequences of which are set forth in SEQ ID NO: 14, SEQ ID NO: 23 and SEQ ID NO: 16, respectively; and
5) an anti-ANGPTL3 antibody or an antigen-binding fragment thereof, comprising an antibody heavy chain variable region and an antibody light chain variable region, wherein the amino acid sequence of the heavy chain variable region is set forth in SEQ ID NO: 9, and the amino acid sequence of the light chain variable region is set forth in SEQ ID NO: 10.

The pharmaceutical composition described in the present disclosure already has sufficient stability for being prepared into a drug and can be stored stable for a long time. The pharmaceutical composition of the present disclosure at least has the advantage of having good stability, good lyophilization morphology, etc.

In some embodiments, the pharmaceutical composition remains stable at 2-8 °C for at least 3 months, at least 6 months, at least 12 months, at least 18 months, or at least 24 months. In some embodiments, the pharmaceutical composition remains stable at 25 °C for at least 3 months, at least 6 months, at least 12 months, at least 18 months, or at least 24 months. In some embodiments, the pharmaceutical composition remains stable at 40 °C for at least 7 days, at least 14 days, or at least 28 days.

In some embodiments, the pharmaceutical composition remains stable after being shaken at 200 rpm at room temperature for at least 12 hours, at least 24 hours, at least 36 hours, at least 48 hours, or at least 60 hours.

In some embodiments, the pharmaceutical composition remains stable after at least 1, at least 2, at least 3, at least 4, at least 5, or at least 6 freeze-thaw (-35 °C/room temperature) cycles.

In some embodiments, the pharmaceutical composition remains stable after being exposed to light (5000 Lx) at 25 °C for at least 1 day, at least 2 days, at least 3 days, at least 4 days, at least 5 days, at least 6 days, at least 7 days, at least 8 days, at least 9 days, at least 10 days, at least 11 days, or at least 12 days.

The present disclosure further provides a method for preparing the aforementioned pharmaceutical composition, comprising the step of subjecting a stock solution of the anti-ANGPTL3 antibody or the antigen-binding fragment thereof to a buffer exchange, wherein the buffer may be an acetic acid-sodium acetate buffer.

In some embodiments, the aforementioned pharmaceutical composition is a liquid formulation. In some other embodiments, the aforementioned pharmaceutical composition is a lyophilized formulation.

The present disclosure further provides a method for preparing a lyophilized formulation comprising an anti-ANGPTL3 antibody or an antigen-binding fragment thereof, comprising the step of lyophilizing the aforementioned pharmaceutical composition. In alternative embodiments, the lyophilizing comprises the steps of pre-freezing, primary drying and secondary drying in sequence.

The present disclosure further provides a lyophilized formulation comprising an anti-ANGPTL3 antibody or an antigen-binding fragment thereof, prepared by the aforementioned method for preparing a lyophilized formulation.

The present disclosure further provides a method for preparing a reconstituted solution of a lyophilized formulation comprising an anti-ANGPTL3 antibody or an antigen-binding fragment thereof, comprising the step of reconstituting the aforementioned lyophilized formulation, wherein the solution used for the reconstitution is selected from the group consisting of, but is not limited to, water for injection, normal saline, and glucose solution. The present disclosure further provides a reconstituted solution comprising an anti-ANGPTL3 antibody or an antigen-binding fragment thereof, prepared by the aforementioned method for preparing a reconstituted solution.

The present disclosure provides an article of manufacture, a product or a kit, comprising any of the aforementioned pharmaceutical compositions, liquid formulations, lyophilized formulations or reconstituted solutions comprising an anti-ANGPTL3 antibody or an antigen-binding fragment thereof of the present disclosure. Alternatively, the article of manufacture, the product or the kit further comprises one or more containers, wherein the containers contain any of the aforementioned pharmaceutical compositions, liquid formulations, lyophilized formulations or reconstituted solutions of the present disclosure. In some embodiments, the containers are glass vials, for example, injection vials made of neutral borosilicate glass tubes.

The present disclosure further provides an article of manufacture, comprising a container, wherein the container contains the aforementioned pharmaceutical composition or lyophilized formulation, or a reconstituted solution of the lyophilized formulation.

The present disclosure further provides a pharmaceutical composition or a lyophilized formulation, or a reconstituted solution of the lyophilized formulation, for use in methods for treating and preventing diseases or disorders.

The present disclosure further provides use of the aforementioned pharmaceutical composition or lyophilized formulation, or a reconstituted solution of the lyophilized formulation, in the preparation of a medicament for treating and/or preventing diseases or disorders.

The present disclosure further provides a method for treating and/or preventing a disease or disorder, comprising administering to a subject in need thereof a therapeutically and/or prophylactically effective amount of the aforementioned pharmaceutical composition or lyophilized formulation, or a reconstituted solution of the lyophilized formulation. In some embodiments, the subject is a human subject.

In some embodiments, the disease or disorder is any disease or disorder associated with ANGPTL3 activity, including ANGPTL3-mediated diseases or disorders.

In some embodiments, examples of the disease or disorder include, but are not limited to, diseases or disorders involving lipid metabolism, such as hyperlipidemia, hyperlipoproteinemia and dyslipidemia. In some embodiments, examples of the disease or disorder include, but are not limited to, atherosclerotic dyslipidemia, diabetic dyslipidemia, hypertriglyceridemia (including severe hypertriglyceridemia, TG > 1000 mg/dL), hypercholesterolemia, chylomicronemia, mixed dyslipidemia (e.g., obesity, metabolic syndrome or diabetes), lipodystrophy, lipoatrophy, and the like. In some embodiments, examples of the disease or disorder may be dyslipidemia caused by decreased LPL activity, LPL deficiency, decreased LDL receptor (LDLR) activity, LDL receptor deficiency (e.g., LDLR^{-/-} homozygous familial hypercholesterolemia), altered ApoC2/ApoE deficiency, increased ApoB, decreased production and/or elimination of very-low-density lipoprotein (VLDL), certain medications (e.g., dyslipidemia caused by glucocorticoid treatment), any genetic predisposition, diet, lifestyles, etc.

In some embodiments, the disease or disorder may be a disease or disorder associated with or caused by hyperlipidemia, hyperlipoproteinemia and/or dyslipidemia, including but not limited to, cardiovascular diseases or disorders such as atherosclerosis, aneurysm, hypertension, angina pectoris, strokes, cerebrovascular diseases, congestive heart failure, coronary artery disease, myocardial infarction, and peripheral vascular disease; acute pancreatitis; nonalcoholic steatohepatitis (NASH); blood glucose abnormalities such as diabetes and obesity.

In some embodiments, other examples of the disease or disorder include cancer/tumors and non-tumor angiogenesis-related diseases or disorders. In alternative embodiments, the disease or disorder may be an ocular angiogenesis-related disease or disorder, such as age-related macular degeneration, central or branch retinal vein occlusion, diabetic retinopathy, or retinopathy of prematurity. In alternative embodiments, the disease or disorder may be an inflammatory disease or disorder, such as arthritis, rheumatoid arthritis (RA), or psoriasis.

### BRIEF DESCRIPTION OF THE DRAWINGS

FIG. 1: the effect of the antibody P8BG on serum triglycerides in SD (Sprague Dawley) rats.
FIG. 2: the effect of anti-ANGPTL3 antibodies on plasma triglycerides in high-fat-diet (HFD) mice.
FIG. 3: the effect of anti-ANGPTL3 antibodies on plasma low-density lipoprotein cholesterol (LDL-C) in HFD mice.
FIG. 4: the effect of anti-ANGPTL3 antibodies on plasma total cholesterol in HFD mice.
FIG. 5: the effect of anti-ANGPTL3 antibodies on plasma triglycerides in APOE mice (apolipoprotein E (ApoE) gene knockout mice).
FIG. 6: the effect of anti-ANGPTL3 antibodies on plasma high-density liptein cholesterol (HDL-C) in APOE mice.
FIG. 7: the pharmacokinetic results of anti-ANGPTL3 antibodies in mice.
FIG. 8: the pharmacokinetic results of anti-ANGPTL3 antibodies in cynomolgus monkeys.

### DETAILED DESCRIPTION

### I. Terminology

In order to facilitate the understanding of the present disclosure, some technical and scientific terms are specifically defined below. Unless otherwise specifically defined herein, all other technical and scientific terms used herein have the meanings generally understood by those of ordinary skill in the art to which the present disclosure pertains.

"Buffer" refers to a buffer that resists changes in pH by the action of its acid-base conjugate components. Examples of buffers that keep the pH within an appropriate range include acetate, succinate, citrate, phosphate, gluconate, histidine salt, oxalate, lactate, phosphate, citrate, tartrate, fumarate, glycylglycine and other organic acid buffers.

"Histidine salt buffer" is a buffer comprising histidine ions. Examples of histidine salt buffers include histidine-hydrochloride buffers, histidine-acetate buffers, histidine-phosphate buffers, histidine-sulfate buffers, and the like, for example, histidine-acetate buffers or histidine-hydrochloride buffers. Histidine-acetate buffers are prepared from histidine and acetic acid, and histidine salt buffers are prepared from histidine and hydrochloric acid.

"Citrate buffer" is a buffer comprising citrate ions. Examples of citrate buffers include citric acid-sodium citrate, citric acid-potassium citrate, citric acid-calcium citrate, citric acid-magnesium citrate, and the like. The citrate buffer may be citric acid-sodium citrate. "Succinate buffer" is a buffer comprising succinate ions. Examples of succinate buffers include succinic acid-sodium succinate, succinic acid-potassium succinate, succinic acid-calcium succinate, and the like. The succinate buffer may be succinic acid-sodium succinate.

"Phosphate buffer" is a buffer comprising phosphate ions. Examples of phosphate buffers include disodium hydrogen phosphate-sodium dihydrogen phosphate, disodium hydrogen phosphate-potassium dihydrogen phosphate, disodium hydrogen phosphate-citric acid, and the like. The phosphate buffer may be disodium hydrogen phosphate-sodium dihydrogen phosphate.

"Acetate buffer" is a buffer comprising acetate ions. Examples of acetate buffers include acetic acid-sodium acetate, acetic acid-histidine salt, acetic acid-potassium acetate, acetic acid-calcium acetate, acetic acid-magnesium acetate, and the like. The acetate buffer may be acetic acid-sodium acetate.

"Pharmaceutical composition" refers to a mixture containing one or more of the compounds or the physiologically/pharmaceutically acceptable salts or prodrugs thereof described herein, and other chemical components, for example, physiologically/pharmaceutically acceptable carriers and excipients. The pharmaceutical composition is intended to promote the administration to an organism, so as to facilitate the absorption of the active ingredient, thereby exerting biological activity. As used herein, "pharmaceutical composition" and "formulation" may be used interchangeably.

Unless otherwise specified, the solvent in the pharmaceutical composition described in the present disclosure in solution form is water.

"Lyophilized formulation" refers to a formulation or a pharmaceutical composition obtained by lyophilizing a pharmaceutical composition or formulation in liquid or solution form *in vacuo.*

The term "about" or "approximately" as used herein means that a numerical value is within an acceptable error range for the particular value determined by one of ordinary skill in the art, and the numerical value depends in part on how the value is measured or determined (i.e., the limits of the measurement system). For example, "about" may mean a standard deviation within 1 or more than 1 in each practice in the art. Or, "about" or "substantially comprise" may mean a range of up to ±20%; for example, a pH of about 5.5 means a pH of 5.5 ± 1.1. Furthermore, particularly for biological systems or processes, the term may mean up to an order of magnitude or up to 5-fold of a numerical value. Unless otherwise stated, when a specific value is provided in the present application and claims, the meaning of "about" or "substantially comprising" should be assumed to be within an acceptable error range for that specific value.

The pharmaceutical composition described in the present disclosure can achieve the effect of being stable: the antibody in the pharmaceutical composition substantially retains its physical and/or chemical stability and/or biological activity after storage; for example, the pharmaceutical composition substantially retains its physical and chemical stability as well as its biological activity after storage. The storage period is generally selected based on a predetermined shelf life of the pharmaceutical composition. There are a variety of analytical techniques currently available for measuring protein stability, and the stability after storage for a selected period of time at a selected temperature can be measured.

A stable pharmaceutical antibody formulation is one in which no significant change is observed under the following conditions: storage at refrigeration temperature (2-8 °C) for at least 3 months, at least 6 months, at least 1 year, or at most 2 years. In addition, stable liquid formulations include liquid formulations that exhibit desirable features after storage at 25 °C for periods including 1 month, 3 months and 6 months, or storage at 40 °C for periods including 1 month. Typical acceptable criteria for stability are as follows: typically, no more than about 10%, for example, no more than about 5%, of antibody monomer is degraded as measured by SEC-HPLC. The pharmaceutical antibody formulation is colorless, or clear to slightly opalescent, by visual analysis. The concentration, pH and osmolality of the formulation have changes of no more than ±10%. Typically, clippings of no more than about 10%, for example, no more than about 5%, are observed. Typically, aggregations of no more than about 10%, for example, no more than about 5%, are formed.

An antibody "retains its physical stability" in a pharmaceutical formulation if it shows no significant increase in aggregation, precipitation and/or denaturation upon visual inspection of color and/or clarity, or as determined by UV light scattering, size exclusion chromatography (SEC), and dynamic light scattering (DLS). Changes in protein conformation can be assessed by fluorescence spectroscopy (which determines the protein tertiary structure) and by FTIR spectroscopy (which determines the protein secondary structure).

An antibody "retains its chemical stability" in a pharmaceutical formulation if it shows no significant chemical change. Chemical stability can be evaluated by detecting and quantifying chemically changed forms of the protein. Degradation processes that often change the chemical structure of proteins include hydrolysis or clipping (assessed by methods such as size exclusion chromatography and SDS-PAGE), oxidation (assessed by methods such as peptide mapping in combination with mass spectroscopy or MALDI/TOF/MS), deamidation (assessed by methods such as ion-exchange chromatography, capillary isoelectric focusing, peptide mapping, and isoaspartic acid determination), and isomerization (assessed by isoaspartic acid content determination, peptide mapping, etc.).

An antibody "retains its biological activity" in a pharmaceutical formulation if the biological activity of the antibody at a given time is within a predetermined range of the biological activity exhibited during the preparation of the pharmaceutical formulation. The biological activity of an antibody can be determined, for example, by an antigen-binding assay.

The three-letter and single-letter codes for amino acids used herein are as described in J. biol. chem, 243, p3558 (1968).

The "antibody" described in the present disclosure refers to an immunoglobulin, and a natural intact antibody is of a tetrapeptide chain structure formed by connection between two identical heavy chains and two identical light chains by interchain disulfide bonds. The heavy chain constant regions of an immunoglobulin differ in their amino acid composition and arrangement, and thus in their antigenicity. Accordingly, immunoglobulins can be divided into five classes, otherwise called isotypes of immunoglobulins, namely IgM, IgD, IgG, IgA and IgE, with their corresponding heavy chains being µ chain, δ chain, γ chain, α chain and ε chain, respectively. Ig of the same class can be divided into different subclasses according to differences in the amino acid composition of the hinge regions and the number and positions of disulfide bonds of the heavy chains; for example, IgG can be divided into IgG1, IgG2, IgG3 and IgG4. Light chains are divided into κ or λ chains according to differences in the constant regions. Each of the five classes of Ig may have a κ chain or λ chain.

In the heavy and light chains of the antibody, the sequences of about 110 amino acids near the N-terminus vary considerably and thus are referred to as variable regions (Fv regions); the remaining amino acid sequences near the C-terminus are relatively stable and thus are referred to as constant regions. The variable regions comprise 3 hypervariable regions (HVRs) and 4 framework regions (FRs) with relatively conservative sequences. The 3 hypervariable regions determine the specificity of the antibody and thus are also known as complementarity determining regions (CDRs). Each light chain variable region (LCVR) or heavy chain variable region (HCVR) consists of 3 CDRs and 4 FRs arranged from the amino-terminus to the carboxyl-terminus in the following order: FR1, CDR1, FR2, CDR2, FR3, CDR3 and FR4. The 3 CDRs of the light chain refer to LCDR1, LCDR2 and LCDR3, and the 3 CDRs of the heavy chain refer to HCDR1, HCDR2 and HCDR3. The CDR amino acid residues of the LCVRs and HCVRs of the antibodies or antigen-binding fragments described in the present disclosure accord with the known Kabat numbering scheme (LCDR1-3, HCDR2-3), or accord with the Kabat and Chothia numbering schemes (HCDR1), in quantitative and positional terms.

The antibody of the present disclosure includes murine antibodies, chimeric antibodies and humanized antibodies, for example, humanized antibodies.

The term "murine antibody", as used in the present disclosure, refers to a monoclonal antibody against human ANGPTL3, prepared according to the knowledge and skill in the art. During the preparation, the ANGPTL3 antigen is injected into a test subject, and then hybridomas expressing antibodies with the desired sequences or functional properties are isolated. In one alternative embodiment of the present disclosure, the murine anti-ANGPTL3 antibody or the antigen-binding fragment thereof may further comprise a light chain constant region of a murine κ or λ chain or a variant thereof, or further comprise a heavy chain constant region of a murine IgG1, IgG2, IgG3 or IgG4 or a variant thereof. The term "chimeric antibody" described herein refers to an antibody obtained by fusing a variable region of a heterologous (e.g., murine) antibody and a constant region of an antibody (e.g., a human antibody), which can alleviate an immune response induced by the heterologous antibody. For example, the human-murine chimeric antibody is established by firstly establishing hybridoma secreting murine specific monoclonal antibody, then cloning a variable region gene from the mouse hybridoma cells, cloning a constant region gene of human antibody as required, connecting the mouse variable region gene and the human constant region gene into a chimeric gene, inserting the chimeric gene into an expression vector, and finally expressing chimeric antibody molecules in a eukaryotic system or prokaryotic system. In one alternative embodiment of the present disclosure, the antibody light chain of the anti-ANGPTL3 chimeric antibody further comprises a light chain constant region of human κ and λ chains or variants thereof. The antibody heavy chain of the ANGPTL3 chimeric antibody further comprises a heavy chain constant region of human IgG1, IgG2, IgG3 or IgG4 or a variant thereof, for example, a heavy chain constant region of human IgG1, IgG2 or IgG4, or an IgG1, IgG2 or IgG4 variant using an amino acid mutation (e.g., an L234A and/or L235A mutation, and/or an S228P mutation).

The term "humanized antibody", also known as a CDR-grafted antibody, refers to an antibody produced by grafting heterologous (e.g., murine) CDR sequences into a human antibody variable region framework, i.e., a different type of human germline antibody framework sequence. Such an antibody can overcome the heterogeneous reaction induced by the chimeric antibody because of carrying a large amount of mouse protein components. Such framework sequences can be obtained from public DNA databases or published references that include germline antibody gene sequences. For example, germline DNA sequences of genes of the human heavy and light chain variable regions can be found in the "VBase" human germline sequence database (available at the Internet address www.mrccpe.com.ac.uk/vbase), as well as in Kabat, E. A. et al., 1991 Sequences of Proteins of Immunological Interest, 5th edition. To avoid the decrease in activity caused by the decrease in immunogenicity, the FR sequence in the human antibody variable region can be subjected to minimum reverse mutation or back mutation to maintain activity. The humanized antibody of the present disclosure also includes humanized antibodies that were further subjected to CDR affinity maturation mutation by yeast display.

The terms "human antibody" and "human-derived antibody" are used interchangeably and mean that one or more of the variable and constant regions are derived from human immunoglobulin sequences. One alternative way is that all of the variable and constant regions are derived from human immunoglobulin sequences, i.e., "fully human-derived antibodies" or "fully human antibodies". These antibodies can be obtained in a variety of ways, including antibodies obtained by isolating B cells from human PBMC, spleen and lymph node tissue and constructing natural single-stranded phage human antibody library by using phage display technology, or by immunizing transgenic mice capable of expressing human antibody light and heavy chains and screening. The human antibodies of the present disclosure also include antibodies that still bind to the antigen of interest and are obtained by mutation of one or more amino acids on the basis of human antibodies.

Besides full-length antibodies, the "antibodies" described herein also include antigen-binding fragments capable of binding to an antigen.

The term "antigen-binding fragment" or "functional fragment" refers to one or more fragments of the antibody that retain the ability to specifically bind to an antigen (e.g., ANGPTL3). It is shown that a fragment of a full-length antibody can be used to perform the antigen-binding function of the antibody. Examples of the binding fragment included in the term "antigen-binding fragment" include (i) Fab fragment, a monovalent fragment consisting of VL, VH, CL and CH1 domains; (ii) F(ab')₂ fragment, a bivalent fragment comprising two Fab fragments linked by a disulfide bridge in the hinge region; (iii) Fd fragment, consisting of VH and CH1 domains; (iv) Fv fragment, consisting of VH and VL domains of one arm of the antibody; (V) dsFv, a stable antigen-binding fragment formed by VH and VL via interchain disulfide bonds therebetween; (vi) diabody, bispecific antibody and multi-specific antibody, comprising such fragments as scFv, dsFv and Fab. In addition, although the two domains of the Fv fragment, VL and VH, are encoded by separate genes, they can be linked by a synthetic linker by a recombination method, thus producing a single protein chain in which the VL and VH regions pair to form a monovalent molecule (referred to as single-chain Fv (scFv); see, e.g., Bird et al. (1988) Science 242:423-426; and Huston et al. (1988) Proc. Natl. Acad. Sci USA 85:5879-5883). Such single-chain antibodies are also included in the term "antigen-binding fragment" of an antibody. Such antibody fragments are obtained by conventional techniques known to those skilled in the art, and screened for utility in the same manner as for intact antibodies. Antigen-binding moieties may be produced by a recombinant DNA technique or by enzyme catalysis or chemical cleavage of intact immunoglobulins. Antibodies may be of different isotypes, e.g., IgG (e.g., subtype IgG1, IgG2, IgG3 or IgG4), IgA1, IgA2, IgD, IgE or IgM antibody.

The "mutations" in the mutated sequences described in the present disclosure include, but are not limited to, "back mutation", "conservative modification" or "conservative replacement or substitution". The "conservative modification" or "conservative replacement or substitution" described in the present disclosure refers to replacement of amino acids in a protein with other amino acids having similar characteristics (e.g., charge, side-chain size, hydrophobicity/hydrophilicity, or backbone conformation and rigidity), so that changes can be frequently made without changing the biological activity of the protein. Those skilled in the art know that, generally speaking, a single amino acid replacement in a non-essential region of a polypeptide does not substantially change the biological activity (see, e.g., Watson et al. (1987) Molecular Biology of the Gene, The Benjamin/Cummings Pub. Co., page 224, (4th edition)). In addition, the replacement of amino acids with similar structure or function is unlikely to disrupt the biological activity. The "mutated sequence" described in the present disclosure refers to a nucleotide sequence and/or amino acid sequence having a different degree of percent sequence identity to the nucleotide sequence and/or amino acid sequence of the present disclosure, when mutational modifications such as replacements, insertions or deletions are appropriately made to the nucleotide sequence and/or amino acid sequence of the present disclosure. The sequence identity described in the present disclosure may be at least 85%, 90% or 95%, for example, at least 95%. Non-limiting examples include 85%, 86%, 87%, 88%, 89%, 90%, 91%, 92%, 93%, 94%, 95%, 96%, 97%, 98%, 99%, and 100%. Sequence comparison and percent identity determination between two sequences can be performed by the default settings for the BLASTN/BLASTP algorithm available on the website National Center For Biotechnology Institute.

"Sequence identity" or "sequence homology", when applied to amino acid sequences, refers to sequence similarity between two proteins or polypeptides. When a position in both of the two sequences to be compared is occupied by the same amino acid residue, e.g., if a position in both polypeptides is occupied by the same amino acid residue, the molecules are identical at that position. Examples of algorithms suitable for determining percent sequence identity and percent sequence similarity are the BLAST and BLAST 2.0 algorithms, which are described in Altschul et al. (1990) J. Mol. Biol. 215:403-410 and Altschul et al.(1977) Nucleic Acids Res. 25:3389-3402, respectively. Software for performing BLAST analyses is publicly available at the National Center for Biotechnology Information (http://www.ncbi.nlm.nih.gov/).

Methods for producing and purifying antibodies and antigen-binding fragments are well known in the prior art, for example, those described in chapters 5-8 and 15 of "Antibodies: A Laboratory Manual", Cold Spring Harbor Press. The antibody or the antigen-binding fragment described herein is genetically engineered to contain one or more human FRs in the non-human CDRs. Human FR germline sequences can be obtained at the website http://imgt.cines.fr of ImMunoGeneTics (IMGT) or from the immunoglobulin journal, 2001ISBN012441351, by comparing the IMGT human antibody variable region germline gene database with the MOE software.

The engineered antibody or antigen-binding fragment of the present disclosure can be prepared and purified by conventional methods. For example, cDNA sequences encoding the heavy and light chains can be cloned and recombined into a GS expression vector. Recombinant immunoglobulin expression vectors can be stably transfected into CHO cells. As a more recommended prior art, mammalian expression systems will result in glycosylation of antibodies, particularly at the highly conserved N-terminal site of the Fc region. Stable clones are obtained by expression of the antibody that specifically binds to human CD40. Positive clones are expanded in a serum-free medium of a bioreactor to produce antibodies. The culture medium with the secreted antibody can be purified by conventional techniques. For example, purification is performed using an A or G Sepharose FF column containing an adjusted buffer. Non-specifically bound fractions are washed away. The bound antibody is eluted by the pH gradient method, and the antibody fragments are detected by SDS-PAGE and collected. The antibody can be filtered and concentrated by conventional methods. Soluble mixtures and polymers can also be removed by conventional methods, such as molecular sieves and ion exchange. The resulting product needs to be immediately frozen, e.g., at -70 °C, or lyophilized.

"Giving" and "treating", when applied to animals, humans, experimental subjects, cells, tissues, organs or biological fluids, refer to contact of an exogenous drug, a therapeutic agent, a diagnostic agent or a composition with the animals, humans, subjects, cells, tissues, organs or biological fluids. "Giving" and "treating" can refer to, for example, therapeutic, pharmacokinetic, diagnostic, research and experimental methods. The treatment of cells comprises contacting the reagent with the cells and contacting the reagent with fluid, where the fluid is in contact with the cells. "Giving" and "treating" also refer to treating, e.g., cells by reagents, diagnosis, binding compositions or by another cell *in vitro* and *ex vivo.* "Treating", when applied to humans, veterinary or research subjects, refers to therapeutic treatment, preventive or prophylactic measures, and research and diagnostic applications.

"Treatment" refers to administration of a therapeutic agent, e.g., a composition comprising any of the binding compositions of the present disclosure, either internally or externally to a subject having one or more disease symptoms for which the therapeutic agent is known to have a therapeutic effect. Typically, the therapeutic agent is administered in an amount effective to alleviate one or more symptoms of the disease in the treated subject or population to induce regression of such symptoms or to inhibit the development of such symptoms to any clinically measurable degree. The amount of therapeutic agent effective to alleviate any particular symptom of the disease (also referred to as the "therapeutically effective amount") may vary depending on factors such as the disease state, age and weight of the subject, and the ability of the drug to produce a desired therapeutic effect in the subject. Whether a symptom of a disease has been alleviated can be evaluated by any clinical testing methods commonly used by doctors or other health care professionals to evaluate the severity or progression of the symptom. Although embodiments of the present disclosure (e.g., treatment methods or articles of manufacture) may be ineffective in alleviating the symptoms of each disease of interest, they shall alleviate the symptoms of the disease of interest in a statistically significant number of subjects as determined by any statistical test method known in the art, such as the Student's t-test, chi-square test, U-test by Mann and Whitney, Kruskal-Wallis test (H-test), Jonckheere-Terpstra test and Wilcoxon test.

"Effective amount" comprises an amount sufficient to ameliorate or prevent a symptom or disorder of a medical disease. An effective amount also refers to an amount sufficient to allow or facilitate diagnosis. The effective amount for a particular subject or veterinary subject may vary depending on factors such as the disorder to be treated, the general health of the subject, the method and route and dosage of administration, and the severity of side effects. An effective amount may be the maximum dosage or administration regimen to avoid significant side effects or toxic effects.

"Tm value" refers to the thermal denaturation temperature of the protein, i.e., the temperature at which half of the proteins are unfolded and the spatial structure of the protein is destroyed. Therefore, the higher the Tm value, the higher the thermal stability of the protein.

### Examples

The present disclosure is further described below with reference to examples, which, however, are not intended to limit the present disclosure. The experimental methods in the examples of the present disclosure in which specific conditions are not specified are generally performed under conventional conditions such as Antibodies: A Laboratory Manual and Molecular Cloning: A Laboratory Manual by Cold Spring Harbor Laboratory, or under conditions recommended by the manufacturer of the raw material or the goods. Reagents without specific origins indicated are commercially available conventional reagents.

### Example 1: Design and Expression of ANGPTL3 Antigens

Human ANGPTL3 protein (Uniprot No. Q9Y5C1), mouse ANGPTL3 protein (Uniprot No. Q9R182), cynomolgus monkey ANGPTL3 protein (Uniprot No. A0A2K5UDC5) and rat ANGPTL3 protein (Uniprot No. F7FHP0) were used as templates of ANGPTL3 in the present disclosure for designing antigens and proteins for detection involved in the present disclosure. The ANGPTL3 proteins were fused with different tags and cloned into pTT5 vectors (Biovector, Cat#: 102762), and 293 cells were transiently transfected with the resulting vectors and underwent expression. The expression products were then purified to give the encoded antigens and proteins for detection of the present disclosure.
1. Full-length human ANGPTL3: human-ANGPTL3(17-460)-His, used for immunization and detection and having the following amino acid sequence: 
MEFGLSWLFLVAILKGVQCSRIDQDNSSFDSLSPEPKSRFAMLDDVKILANGL
LQLGHGLKDFVHKTKGQINDIFQKLNIFDQSFYDLSLQTSEIKEEEKELRRT
TYKLQVKNEEVKNMSLELNSKLESLLEEKILLQQKVKYLEEQLTNLIQNQP
 
ETPEHPEVTSLKTFVEKQDNSIKDLLQTVEDQYKQLNQQHSQIKEIENQLR
RTSIQEPTEISLSSKPRAPRTTPFLQLNEIRNVKHDGIPAECTTIYNRGEHTSG
MYAIRPSNSQVFHVYCDVISGSPWTLIQHRIDGSQNFNETWENYKYGFGRL
DGEFWLGLEKIYSIVKQSNYVLRIELEDWKDNKHYIEYSFYLGNHETNYTL
HLVAITGNVPNAIPENKDLVFSTWDHKAKGHFNCPEGYSGGWWWHDECG
ENNLNGKYNKPRAKSKPERRRGLSWKSQNGRLYSIKSTKMLIHPTDSESFE
HHHHHHHHHH
SEQ ID NO: 1
 Note: The single-underlined part represents the signal peptide sequence, the italicized part represents the His sequence, and the part in bold type represents the amino acids from positions 17 to 460 of the full-length human ANGPTL3 protein.
2. Extracellular region of human ANGPTL3: human ANGPTL3(17-170)-Flag-His, used for immunization and detection and having the following amino acid sequence: 
MEFGLSWLFLVAILKGVQCSRIDQDNSSFDSLSPEPKSRFAMLDDVKILANGL
LQLGHGLKDFVHKTKGQINDIFQKLNIFDQSFYDLSLQTSEIKEEEKELRRT
TYKLQVKNEEVKNMSLELNSKLESLLEEKILLQQKVKYLEEQLTNLIQNQP
ETPEHPEVTSLKTFVEKGSSDYKDDDDKHHHHHH
SEQ ID NO: 2
 Note: The single-underlined part represents the signal peptide, the double-underlined part represents the linking peptide, the italicized part represents the Flag and His, and the part in bold type represents the amino acids from positions 17 to 170 of the full-length human ANGPTL3 protein.
3. Fusion protein of the extracellular region of human ANGPTL3 (human ANGPTL3(17-170)) and the mouse IgG2aFc segment (abbreviated as mFc): human ANGPTL3(17-170)-mFc, used for detection and having the following amino acid sequence: 
MEFGLSWLFLVAILKGVQCSRIDQDNSSFDSLSPEPKSRFAMLDDVKILANGL
LQLGHGLKDFVHKTKGQINDIFQKLNIFDQSFYDLSLQTSEIKEEEKELRRT
TYKLQVKNEEVKNMSLELNSKLESLLEEKILLQQKVKYLEEQLTNLIQNQP
ETPEHPEVTSLKTFVEKEPRGPTIKPCPPCKCPAPNLLGGPSVFIFPPKIKDVLMIS
LSPIVTCVVVDVSEDDPDVQISWFVNNVEVHTAQTQTHREDYNSTLRVVSALPIQHQ
DWMSGKEFKCKVNNKDLPAPIERTISKPKGSVRAPQVYVLPPPEEEMTKKQVTLTC
MVTDFMPEDIYVEWTNNGKTELNYKNTEPVLDSDGSYFMYSKLRVEKKNWVERNSY
SCSVVHEGLHNHHTTKSFSRTPGK
SEQ ID NO: 3
 Note: The underlined part represents the signal peptide, the italicized part represents mouse IgG2aFc, and the part in bold type represents the amino acids from positions 17 to 170 of the full-length human ANGPTL3 protein.
4. Fusion protein of the extracellular region of human ANGPTL3 (human ANGPTL3(17-220)) and the mouse IgG2aFc segment: human ANGPTL3(17-220)-mFc, used for immunization and detection and having the following amino acid sequence: 
MEFGLSWLFLVAILKGVQCSRIDQDNSSFDSLSPEPKSRFAMLDDVKILANGL
LQLGHGLKDFVHKTKGQINDIFQKLNIFDQSFYDLSLQTSEIKEEEKELRRT
TYKLQVKNEEVKNMSLELNSKLESLLEEKILLQQKVKYLEEQLTNLIQNQP
ETPEHPEVTSLKTFVEKQDNSIKDLLQTVEDQYKQLNQQHSQIKEIENQLR
RTSIQEPTEISLSSKPEPRGPTIKPCPPCKCPAPNLLGGPSVFIFPPKIKDVLMISLSP
IVTCVVVDVSEDDPDVQISWFVNNVEVHTAQTQTHREDYNSTLRVVSALPIQHQDW
MSGKEFKCKVNNKDLPAPIERTISKPKGSVRAPQVYVLPPPEEEMTKKQVTLTCMVT
DFMPEDIYVEWTNNGKTELNYKNTEPVLDSDGSYFMYSKLRVEKKNWVERNSYSCS
VVHEGLHNHHTTKSFSRTPGK
SEQ ID NO: 4
 Note: The underlined part represents the signal peptide, the italicized part represents mouse IgG2aFc, and the part in bold type represents the amino acids from positions 17 to 220 of the full-length human ANGPTL3 protein.
5. Full-length mouse ANGPTL3: mouse ANGPTL3(17-455)-His, used for immunization and detection and having the following amino acid sequence: 
MEFGLSWLFLVAILKGVQCSRVDPDLSSFDSAPSEPKSRFAMLDDVKILANGL
LQLGHGLKDFVHKTKGQINDIFQKLNIFDQSFYDLSLRTNEIKEEEKELRRT
TSTLQVKNEEVKNMSVELNSKLESLLEEKTALQHKVRALEEQLTNLILSPA
GAQEHPEVTSLKSFVEQQDNSIRELLQSVEEQYKQLSQQHMQIKEIEKQLR
KTGIQEPSENSLSSKSRAPRTTPPLQLNETENTEQDDLPADCSAVYNRGEHT
SGVYTIKPRNSQGFNVYCDTQSGSPWTLIQHRKDGSQDFNETWENYEKGF
GRLDGEFWLGLEKIYAIVQQSNYILRLELQDWKDSKHYVEYSFHLGSHETN
YTLHVAEIAGNIPGALPEHTDLMFSTWNHRAKGQLYCPESYSGGWWWNDI
CGENNLNGKYNKPRTKSRPERRRGIYWRPQSRKLYAIKSSKMMLQPTTHH
HHHHHHHH
SEQ ID NO: 5
 Note: The underlined part represents the signal peptide, the italicized part represents the His sequence, and the part in bold type represents the amino acids from positions 17 to 455 of the full-length mouse ANGPTL3 protein.
6. Fusion protein of the extracellular region of mouse ANGPTL3 (mouse-ANGPTL3(17-220)) and the mouse IgG2aFc segment: mouse ANGPTL3(17-220)-mFc, used for immunization and having the following amino acid sequence: 
MEFGLSWLFLVAILKGVQCSRVDPDLSSFDSAPSEPKSRFAMLDDVKILANGL
LQLGHGLKDFVHKTKGQINDIFQKLNIFDQSFYDLSLRTNEIKEEEKELRRT
TSTLQVKNEEVKNMSVELNSKLESLLEEKTALQHKVRALEEQLTNLILSPA
GAQEHPEVTSLKSFVEQQDNSIRELLQSVEEQYKQLSQQHMQIKEIEKQLR
KTGIQEPSENSLSSKSEPRGPTIKPCPPCKCPAPNLLGGPSVFIFPPKIKDVLMISLS
PIVTCVVVDVSEDDPDVQISWFVNNVEVHTAQTQTHREDYNSTLRVVSALPIQHQDW
 
MSGKEFKCKVNNKDLPAPIERTISKPKGSVRAPQVYVLPPPEEEMTKKQVTLTCMVT
DFMPEDIYVEWTNNGKTELNYKNTEPVLDSDGSYFMYSKLRVEKKNWVERNSYSCS
VVHEGLHNHHTTKSFSRTPGK
SEQ ID NO: 6
 Note: The underlined part represents the signal peptide, the italicized part represents mouse IgG2aFc, and the part in bold type represents the amino acids from positions 17 to 220 of the full-length mouse ANGPTL3 protein.
7. Full-length cynomolgus monkey ANGPTL3: cynomolgus monkey ANGPTL3(17-460)-His, used for detection and having the following amino acid sequence: 
MEFGLSWLFLVAILKGVQCSRIDQDNSSFDSVSPEPKSRFAMLDDVKILANGL
LQLGHGLKDFVHKTKGQINDIFQKLNIFDQSFYDLSLQTSEIKEEEKELRRT
TYKLQVKNEEVKNMSLELNSKLESLLEEKILLQQKVKYLEEQLTNLIQNQP
ATPEHPEVTSLKSFVEKQDNSIKDLLQTVEEQYKQLNQQHSQIKEIENQLR
MTNIQEPTEISLSSKPRAPRTTPFLQLNEIRNVKHDGIPADCTTIYNRGEHIS
GTYAIRPSNSQVFHVYCDVVSGSPWTLIQHRIDGSQNFNETWENYKYGFGR
LDGEFWLGLEKIYSIVKQSNYVLRIELEDWKDNKHYIEYSFYLGNHETNYT
LHVVKITGNVPNAIPENKDLVFSTWDHKAKGHFSCPESYSGGWWWHDEC
GENNLNGKYNKPRTKSKPERRRGLSWKSQNGRLYSIKSTKMLIHPTDSESF
EHHHHHHHHHH
SEQ ID NO: 7
 Note: The underlined part represents the signal peptide, the italicized part represents the His sequence, and the part in bold type represents the amino acids from positions 17 to 460 of the full-length cynomolgus monkey ANGPTL3 protein.
8. Full-length rat ANGPTL3: rat ANGPTL3(17-455)-His, used for detection and having the following amino acid sequence: 
MEFGLSWLFLVAILKGVQCSRVDPDLSPFDSVPSEPKSRFAMLDDVKILANGL
LQLGHGLKDFVHKTKGQINDIFQKLNIFDQCFYDLSLQTNEIKEEEKELRR
TTSKLQVKNEEVKNMSLELNSKLESLLEEKMALQHRVRALEEQLTSLVQN
PPGAREHPEVTSLKSFVEQQDNSIRELLQSVEEQYKQLSQQHIQIKEIENQL
RKTGIQEPTENSLYSKPRAPRTTPPLHLKEAKNIEQDDLPADCSAIYNRGEH
TSGVYTIRPSSSQVFNVYCDTQSGTPRTLIQHRKDGSQNFNQTWENYEKGF
GRLDGEFWLGLEKIYAIVKQSNYILRLELQDWKDSKHYAEYSFHLGNHET
NYTLHVAEIAANIPEALPEHRDLMFSTWDHRAKGQLYCPESYSGGWWFSD
MCGENNLNGKYNKPRAKSKPERRRGISWRPRGGKLYSIKSSKMMLQPTTH
HHHHHHHHH
SEQ ID NO: 8
 Note: The underlined part represents the signal peptide, the italicized part represents the His sequence, and the part in bold type represents the amino acids from positions 17 to 455 of the full-length rat ANGPTL3 protein.

### Example 2: Purification of ANGPTL3 Antigen Proteins, Hybridoma Antibodies and Recombinant Antibodies

### 2.1. Purification of recombinant proteins with His-tags or recombinant proteins with Flag-his tags

A cell expression sample was centrifuged at high speed to remove cells and insoluble impurities, the supernatant was collected, and imidazole was added until the final concentration was 5 mM. A nickel column was equilibrated with a PBS solution containing 5 mM imidazole and washed with 2-5 column volumes. The cell supernatant was loaded onto the column. The column was washed with a PBS solution containing 5 mM imidazole until the A280 reading dropped to the baseline. The chromatography column was then washed with PBS + 10 mM imidazole to remove non-specifically bound protein impurities, and the flow-through was collected. The target protein was eluted with a PBS solution containing 300 mM imidazole and the elution peak was collected.

The collected eluate was further purified using a Superdex gel filtration column and then collected with tubes. After the obtained sample was confirmed by SDS-PAGE, peptide mapping and LC-MS, it was aliquoted for later use. Thus, the recombinant proteins with His tags or the recombinant proteins with Flag-his tags were obtained.

### 2.2. Purification of hybridoma antibodies or recombinant proteins with mFc tags

A sample was centrifuged at high speed to remove cells and insoluble impurities, and the supernatant was collected. A Protein A affinity column was equilibrated with 1× PBS and washed with 2-5 column volumes. The cell expression supernatant sample obtained by centrifugation was loaded onto the column. The column was washed with 1× PBS until the A280 reading dropped to the baseline. The column was washed with 1× PBS. The target protein was eluted with 0.1 M acetic acid (pH 3.5-4.0), and the eluted protein was collected, then immediately made neutral with 1 M Tris-HCl (pH 7.0), and finally exchanged into 1× PBS buffer by dialysis or concentration. The sample was collected, confirmed by electrophoresis, peptide mapping and LC-MS, and then aliquoted for later use. Thus, the recombinant proteins with mFc tags or the hybridoma antibodies were obtained.

### 2.3. Purification of antibodies and recombinant proteins with Fc tags

A sample was centrifuged at high speed to remove cells and insoluble impurities, and the supernatant was collected. A Protein G affinity column was equilibrated with 1× PBS and washed with 2-5 column volumes. The cell expression supernatant sample obtained by centrifugation was loaded onto the column. The column was washed with 1× PBS until the A280 reading dropped to the baseline. The column was washed with 1× PBS. The target protein was eluted with acetic acid (pH 3.0), and the eluted protein was collected, then immediately made neutral with 1 M Tris-HCl (pH 7.0), and finally exchanged into 1× PBS buffer by dialysis or concentration. The sample was collected, confirmed by electrophoresis, peptide mapping and LC-MS, and then aliquoted for later use. Thus, the antibodies or the recombinant proteins with hFc tags were obtained.

### Example 3: Preparation of Anti-ANGPTL3 Antibodies

### 3.1. Screening for anti-ANGPTL3 human-derived antibodies by phage library

A human phage single-chain antibody library was packaged and concentrated, and then the phage library (10¹²-10¹³/pfu) was suspended in 1 mL of 2% MPBS (PBS containing 2% skim milk powder), and 100 µL of Dynabeads^{®} M-280 streptavidin (Invitrogen, Cat No. 11206D) was added. The tube was placed on a rotating plate and inverted repeatedly, and then blocked at room temperature for 1 h. The tube was left on a magnetic rack for 2 min, the Dynabeads were removed, and the phage library was transferred to a new tube. 2 µg/mL biotin-labeled human ANGPTL3(17-460)-His was added to the blocked phage library, and the tube was placed on a rotating plate and inverted repeatedly for 1 h. Meanwhile, 100 µL of Dynabeads were measured out and suspended in 1 mL of 2% MPBS, and the tube was placed on a rotating plate and inverted repeatedly, and blocked at room temperature for 1 h. The tube was left on a magnetic rack for 2 min, and the blocking solution was pipetted off. The blocked Dynabeads were added to the mixture of the phage library and human ANGPTL3(17-460)-His, and the tube containing the resulting mixture was placed on a rotating plate and inverted repeatedly for 15 min. The tube was left on a magnetic rack for 2 min, and the mixture was pipetted off. Dynabeads were washed 10 times with 1 mL of PBST (PBS containing 0.1% Tween-20), and 0.5 mL of 1 mg/mL trypsin (Sigma, Cat No. T1426-250MG) was added. The tube was placed on a rotating plate and incubated for 15 min, inverted repeatedly, and elution was performed. The eluted phage was used to infect TG1 *E. coli,* and the TG1 *E. coli* was plated. Single clones were randomly picked for phage ELISA.

The clones were seeded into a 96-well deep-well plate (Nunc Cat No. 260251) and incubated at 37 °C for 16-18 h. A small number of the incubated clones were taken and seeded into another 96-well deep-well plate. When the OD600 reached about 0.5, M13K07 helper phages (NEB, Cat No. N0315S) were added for packaging. The mixture was centrifuged at 4000 g for 10 min to remove bacteria, and the culture solution was collected using a pipette and subjected to an ANGPTL3 binding ELISA assay. The positive clone strains were cryopreserved and sent to a sequencing company for sequencing. The amino acid sequences corresponding to the positive clone P8 are as follows:
> Heavy chain variable region sequence of P8: 
QVQLVQSGAEVKKPGASVKVSCKASGYTFTSYDINWVRQAPGQGLEWVGLINPRD
DSTSYAQKFQGRVTMTRDTSTSTMYMELSSLRSEDTAVYFCARDLGSIREVLYYGM
DVWGQGTTVTVSS
SEQ ID NO: 9
> Light chain variable region sequence of P8:

DVVMTQSPLSLPVTPGEPASISCRSSQSLLHSNGYTYLDWYLQKPGQSPQLLIYLGS

NRASGVPDRFSGSGSGTDFTLKISRVEAEDVGVYYCMQALQTPLTFGQGTRLEIK
SEQ ID NO: 10

Note: The underlined parts in the sequences represent the CDR sequences determined according to the Kabat numbering system, and the italicized parts represent FR sequences.

### 3.2. Mutation of anti-ANGPTL3 antibody

Based on the three-dimensional structure of the P8 antibody, the amino acid residue at position 55 (naturally sequentially numbered) in the heavy chain variable region of P8 was mutated from D to E, and the amino acid residue at position 34 (naturally sequentially numbered) in the light chain variable region of P8 was mutated from G to V, thus obtaining a novel antibody molecule P8B, wherein the amino acid sequence of P8B is as follows:
> Heavy chain variable region sequence of P8B: 
QVQLVQSGAEVKKPGASVKVSCKASGYTFTSYDINWVRQAPGQGLEWVGLINPRED
STSYAQKFQGRVTMTRDTSTSTMYMELSSLRSEDTAVYFCARDLGSIREVLYYGMD
VWGQGTTVTVSS
SEQ ID NO: 17
> Light chain variable region sequence of P8B:

DVVMTQSPLSLPVTPGEPASISCRSSQSLLHSNVYTYLDWYLQKPGQSPQLLIYLGS
NRASGVPDRFSGSGSGTDFTLKISRVEAEDVGVYYCMQALQTPLTFGQGTRLEIK
SEQ ID NO: 18

Note: The underlined parts in the sequences represent the CDR sequences determined according to the Kabat numbering system, and the italicized parts represent FR sequences.

**Table 1. The CDR sequences of the P8B antibody molecule**

| Light chain CDR | | Heavy chain CDR | |
|---|---|---|---|
| LCDR1 | RSSQSLLHSNVYTYLD (SEQ ID NO: 11) | HCDR1 | SYDIN (SEQ ID NO: 14) |
| LCDR2 | LGSNRAS (SEQ ID NO: 12) | HCDR2 | LINPREDSTSYAQKFQG (SEQ ID NO: 15) |
| LCDR3 | MQALQTPLT (SEQ ID NO: 13) | HCDR3 | DLGSIREVLYYGMDV (SEQ ID NO: 16) |

### 3.3. Construction and expression of anti-ANGPTL3 antibody

A primer was designed, and a VH/VK gene fragment of the antibody was constructed by PCR and homologously recombined with an expression vector pHr (with a signal peptide and a constant region gene (CH1-FC/CL) fragment). Thus, the antibody full-length expression vector VH-CH1-FC-pHr/VK-CL-pHr was constructed. The heavy chain constant region of the antibody may be selected from the group consisting of the constant regions of human IgG1, IgG2, IgG4 and variants thereof, and the light chain constant region of the antibody may be selected from the group consisting of the light chain constant regions of human κ and λ chains and variants thereof. Illustratively, the antibody heavy chain constant region is selected from a human IgG4-YTE variant, the sequence of which is set forth in SEQ ID NO: 19, and the light chain constant region is selected from the constant region of a human κ chain, the sequence of which is set forth in SEQ ID NO: 20.
> Heavy chain constant region sequence of human IgG4-YTE variant: 
ASTKGPSVFPLAPCSRSTSESTAALGCLVKDYFPEPVTVSWNSGALTSGVHTFPA
VLQSSGLYSLSSVVTVPSSSLGTKTYTCNVDHKPSNTKVDKRVESKYGPPCPPCP
APEFLGGPSVFLFPPKPKDTLYITREPEVTCVVVDVSQEDPEVQFNWYVDGVEV
HNAKTKPREEQFNSTYRVVSVLTVLHQDWLNGKEYKCKVSNKGLPSSIEKTISK
AKGQPREPQVYTLPPSQEEMTKNQVSLTCLVKGFYPSDIAVEWESNGQPENNYK
TTPPVLDSDGSFFLYSRLTVDKSRWQEGNVFSCSVMHEALHNHYTQKSLSLSLG
K
SEQ ID NO: 19
> Light chain constant region sequence of human κ chain:



SEQ ID NO: 20

Illustratively, the light/heavy chain constant regions described above were combined with the aforementioned variable regions of P8B to form a complete anti-ANGPTL3 antibody: P8BG. The light/heavy chain sequences of the antibody are as follows:
> Heavy chain sequence of P8BG: 
QVQLVQSGAEVKKPGASVKVSCKASGYTFTSYDINWVRQAPGQGLEWVGLINPRED
STSYAQKFQGRVTMTRDTSTSTMYMELSSLRSEDTAVYFCARDLGSIREVLYYGMD
VWGQGTTVTVSSASTKGPSVFPLAPCSRSTSESTAALGCLVKDYFPEPVTVSWNS
GALTSGVHTFPAVLQSSGLYSLSSVVTVPSSSLGTKTYTCNVDHKPSNTKVDKR
VESKYGPPCPPCPAPEFLGGPSVFLFPPKPKDTLYITREPEVTCVVVDVSQEDPEV
QFNWYVDGVEVHNAKTKPREEQFNSTYRVVSVLTVLHQDWLNGKEYKCKVS
NKGLPSSIEKTISKAKGQPREPQVYTLPPSQEEMTKNQVSLTCLVKGFYPSDIAVE
WESNGQPENNYKTTPPVLDSDGSFFLYSRLTVDKSRWQEGNVFSCSVMHEALH
NHYTQKSLSLSLGK
SEQ ID NO: 21
> Light chain sequence of P8BG:

DVVMTQSPLSLPVTPGEPASISCRSSQSLLHSNVYTYLDWYLQKPGQSPQLLIYLGS
NRASGVPDRFSGSGSGTDFTLKISRVEAEDVGVYYCMQALQTPLTFGQGTRLEIKR
TVAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGEC
SEQ ID NO: 22

### Example 4: In Vitro Evaluation of Anti-ANGPTL3 Antibodies

### 4.1: ELISA assay for binding of anti-ANGPTL3 antibodies to ANGPTL3 proteins

An anti-ANGPTL3 antibody inhibits or interferes with at least one activity of an ANGPTL3 protein by binding to the ANGPTL3 protein. The binding activity of the anti-ANGPTL3 antibody to the ANGPTL3 antigen was measured by an ELISA assay. The ANGPTL3 protein (human ANGPTL3(17-170)-mFc) was fixed to a 96-well microplate by binding to the goat anti-mouse IgG with which the microplate was coated, and the binding activity of the antibody to ANGPTL3 was determined according to the intensity of the signal after the antibody was added. Meanwhile, ANGPTL3 proteins with HIS tags (mouse ANGPTL3(17-455)-His, cynomolgus monkey ANGPTL3(17-460)-His, and rat ANGPTL3(17-455)-His) labeled with a biotin labeling kit (Dojindo China, LK03) were fixed to a 96-well microplate by binding to streptavidin with which the microplate was coated, and the binding activity of the antibody to ANGPTL3 was determined according to the intensity of the signal after the antibody was added.

Goat anti-mouse IgG (Sigma, M3534-1ML) was diluted to a concentration of 2 µg/mL with a pH 7.4 PBS buffer (Shanghai BasalMedia, B320) and added to a 96-well microplate (Corning, CLS3590-100EA) at 50 µL/well. The plate was left in a 37 °C incubator for 3 h or left at 4 °C overnight (16-18 h). After the liquid was discarded, a PBS-diluted 5% skim milk (BD, 232100) blocking solution was added at 250 µL/well, and the plate was incubated in a 37 °C incubator for 3 h or left at 4 °C overnight (16-18 h) for blocking. After the blocking was complete, the blocking solution was discarded, and after the plate was washed 3 times with PBST buffer (pH 7.4 PBS containing 0.05% Tween-20), human ANGPTL3(17-170)-mFc (the sequence of which is set forth in SEQ ID NO: 3) diluted to 0.5 µg/mL with sample diluent (pH 7.4 PBS containing 1% BSA) was added at 50 µL/well, and the plate was incubated in a 37 °C incubator for 2 h. After the incubation was complete, the reaction solution in the microplate was discarded. After the plate was washed 3 times with PBST, a test antibody that had been diluted with sample diluent to different concentrations was added at 50 µL/well, and the plate was incubated in a 37 °C incubator for 2 h. After the incubation was complete, the plate was washed 3 times with PBST, and a goat anti-human secondary antibody (Jackson Immuno Research, 109-035-003) diluted with sample diluent was added at 50 µL/well. The plate was incubated at 37 °C for 1 h. After the plate was washed 3 times with PBST, TMB chromogenic substrate (KPL, 52-00-03) was added at 50 µL/well. The plate was incubated at room temperature for 2-5 min, and MH₂SO₄ was added at 50 µL/well to stop the reaction. The absorbance at 450 nm was recorded using a VersaMax microplate reader, and the EC₅₀ value for the binding of the anti-ANGPTL3 antibody to the ANGPTL3 antigen protein was calculated. The experimental results are shown in Table 3.

Streptavidin (Sigma, S4762-5MG) was diluted to a concentration of 3 µg/mL with a pH 7.4 PBS buffer (Shanghai BasalMedia, B320) and added to a 96-well microplate (Corning, CLS3590-100EA) at 50 µL/well. The plate was left in a 37 °C incubator for 3 h or left at 4 °C overnight (16-18 h). After the liquid was discarded, a PBS-diluted 5% skim milk (BD, 232100) blocking solution was added at 250 µL/well, and the plate was incubated in a 37 °C incubator for 3 h or left at 4 °C overnight (16-18 h) for blocking. After the blocking was complete, the blocking solution was discarded, and after the plate was washed 3 times with PBST buffer (pH 7.4 PBS containing 0.05% Tween-20), biotin-labeled mouse ANGPTL3(17-455)-His (the sequence of which is set forth in SEQ ID NO: 5), cynomolgus monkey ANGPTL3(17-460)-His (the sequence of which is set forth in SEQ ID NO: 7) and rat ANGPTL3(17-455)-His (the sequence of which is set forth in SEQ ID NO: 8) that had been diluted to 0.5 µg/mL with sample diluent (pH 7.4 PBS containing 1% BSA) were added at 50 µL/well, and the plate was incubated in a 37 °C incubator for 2 h. After the incubation was complete, the reaction solution in the microplate was discarded. After the plate was washed 3 times with PBST, a test antibody that had been diluted with sample diluent to different concentrations was added at 50 µL/well, and the plate was incubated in a 37 °C incubator for 2 h. After the incubation was complete, the plate was washed 3 times with PBST, and an HRP-labeled goat anti-mouse secondary antibody (Jackson Immuno Research, 115-035-003) or a goat anti-human secondary antibody (Jackson Immuno Research, 109-035-003) or a mouse HRP/anti-M13-conjugated secondary antibody (Sino Biological, Cat No. 11973-MM05-100, used for phage screening) that had been diluted with sample diluent was added at 50 µL/well. The plate was incubated at 37 °C for 1 h. After the plate was washed 3 times with PBST, TMB chromogenic substrate (KPL, 52-00-03) was added at 50 µL/well. The plate was incubated at room temperature for 2-5 min, and MH₂SO₄ was added at 50 µL/well to stop the reaction. The absorbance at 450 nm was recorded using a VersaMax microplate reader, and the EC₅₀ value for the binding of the anti-ANGPTL3 antibody to the ANGPTL3 antigen protein was calculated. The experimental results are shown in Table 2. The results show that the anti-ANGPTL3 antibodies of the present disclosure are capable of binding to ANGPTL3 proteins of different species.

**Table 2. The ELISA assay for the binding of the anti-ANGPTL3 antibodies to the ANGPTL3 proteins of different species**

| Antibody | Binding to human ANGPTL3 EC₅₀ (nM) | Binding to mouse ANGPTL3 EC₅₀ (nM) | Binding to cynomolgus monkey ANGPTL3 EC₅₀ (nM) | Binding to rat ANGPTL3 EC₅₀ (nM) |
|---|---|---|---|---|
| P8G | 0.361 | 1.732 | 2.528 | 2.115 |
| P8BG | 0.172 | 0.105 | 0.200 | 0.528 |

### 4.2. HTRF assay for binding of anti-ANGPTL3 antibodies to ANGPTL3 antigen proteins

An HTRF assay was also used to measure the binding activity of the anti-ANGPTL3 antibodies to the antigens. The ANGPTL3 antigens with HIS tags (human ANGPTL3(17-460)-His, mouse ANGPTL3(17-455)-His, cynomolgus monkey ANGPTL3(17-460)-His, and rat ANGPTL3(17-455)-His) labeled with a biotin labeling kit (Dojindo China, LK03) bound to the HTRF reagent Streptavidin-Tb cryptata (Cisbio, 610SATLA), and after an antibody was added, the antibody bound to the HTRF reagent Pab anti-mouse IgG-XI,665 (Cisbio, 61PAMXLA) and angiopoietin-like protein 3. The intensity of the signal was used to determine the binding activity of the antibody to angiopoietin-like protein 3. 4 µg/mL biotin-labeled human ANGPTL3(17-460)-His (the sequence of which is set forth in SEQ ID NO: 1), mouse ANGPTL3(17-455)-His (the sequence of which is set forth in SEQ ID NO: 5), cynomolgus monkey ANGPTL3(17-460)-His (the sequence of which is set forth in SEQ ID NO: 7) and rat ANGPTL3(17-455)-His (the sequence of which is set forth in SEQ ID NO: 8) were prepared using a diluent (pH 7.4 PBS containing 1% BSA) and added to a 384-well plate (PerkinElmer, optiplatte-384) at 5 µL/well, then a mixture of the HTRF reagent Streptavidin-Tb cryptata and Pab anti-mouse IgG-XL665 was added at 5 µL/well, and finally a test antibody that had been diluted with sample diluent to different concentrations was added at 10 µL/well. The plate was incubated at 25 °C for 1 h. Values were recorded using a PHERAstar FS (BMG LabTECH) at the HTRF module, and the EC50 value for the binding of the anti-ANGPTL3 antibody to angiopoietin-like protein 3 was calculated. The experimental results are shown in Table 3. The results show that the antibodies of the present disclosure all have good binding activity to the ANGPTL3 antigens of human, mouse, cynomolgus monkey, rat and other species.

**Table 3. The HTRF assay for the binding of the anti-ANGPTL3 antibodies to the ANGPTL3 proteins**

| Antibody | EC₅₀ (nM) for binding to ANGPTL3 proteins | | | |
|---|---|---|---|---|
| | Human ANGPTL3 | Mouse ANGPTL3 | Cynomolgus monkey ANGPTL3 | Rat ANGPTL3 |
| P8G | 0.784 | 0.450 | 0.303 | 0.484 |
| P8BG | 0.277 | 0.321 | 0.360 | 0.362 |

### 4.3. Enzyme activity assay for inhibition of LPL by anti-ANGPTL3 antibodies

The enzyme activity detection experiment of LPL was used to measure the activity of the anti-ANGPTL3 antibody in blocking the inhibition of the LPL enzyme by ANGPTL3 proteins.

Bovine LPL (Sigma, L2254-5KU) was diluted with a dilution buffer (pH 7.4 PBS + 2 mg/mL BSA) to 12.5 units and added to a 96-well plate (Corning, 3603) at 25 µL/well, then a test antibody that had been diluted with sample diluent to different concentrations was added at 25 µL/well, and then 27.6 µg/mL ANGPTL3 proteins (human ANGPTL3(17-170)-Flag-His (the sequence of which is set forth in SEQ ID NO: 2), mouse ANGPTL3(17-455)-His (the sequence of which is set forth in SEQ ID NO: 5), cynomolgus monkey ANGPTL3(17-460)-His (the sequence of which is set forth in SEQ ID NO: 7), and rat ANGPTL3(17-455)-His (the sequence of which is set forth in SEQ ID NO: 8)) were added at 25 µL/well. The 96-well plate was well shaken on a shaker and incubated in a 37 °C incubator for 30 min. Finally, a 20 µM substrate (Sigma, 30058-10MG-F) that had been diluted with dilution buffer was added at 25 µL/well, and the 96-well plate was well shaken on a shaker and incubated in a 37 °C incubator for 30 min. Readings were taken at Ex535/Em612 using a Flexstation3 microplate reader. The concentrations and corresponding fluorescence data were processed using Graphpad Prism 5 software, and the IC₅₀ was calculated. The experimental results are shown in Table 4. The results show that the antibodies of the present disclosure have good inhibitory effects against the LPL enzyme.

**Table 4. The activity of the anti-ANGPTL3 antibody in blocking the inhibition of LPL by ANGPTL3 of different species**

| | IC₅₀ (nM) | | | |
|---|---|---|---|---|
| Antibody | Human ANGPTL3 | Mouse ANGPTL3 | Cyno ANGPTL3 | Rat ANGPTL3 |
| P8BG | 75.3 | 41.5 | 53.6 | 132.2 |

### 4.4. Biacore assay of anti-ANGPTL3 antibody and ANGPTL3 proteins

Biacore was used to measure the affinities of the test anti-ANGPTL3 antibody for human, monkey, rat and mouse ANGPTL3. The method is as follows:
A Protein A biosensor chip (Cat. # 29127556, GE) was used to affinity-capture a certain amount of test antibody, and then certain concentrations of the human, monkey, rat and mouseANGPTL3 antigens (human-ANGPTL3 (R&D, 3829-AN), mouse ANGPTL3(17-455)-His (the sequence of which is set forth in SEQ ID NO: 5), cynomolgus monkey ANGPTL3(17-460)-His (the sequence of which is set forth in SEQ ID NO: 7), and rat ANGPTL3(17-455)-His (the sequence of which is set forth in SEQ ID NO: 8)) were allowed to flow over the surface of the chip. Reaction signals were detected in real time using a Biacore T200 instrument to obtain binding and dissociation curves. After dissociation was complete in each cycle, the biochip was washed and regenerated with a glycine-hydrochloric acid regeneration solution (Cat. # BR-1003-54, GE) having a pH of 1.5. The running buffer for the experiment was 1× HBS-EP buffer (Cat. # BR-1001-88, GE). Fitting was performed on the data obtained from the experiment using GE Biacore T200 evaluation software (version 3.0) with the (1:1) Langmuir model to obtain affinity values. The experimental results are shown in Table 5. The results show that the anti-ANGPTL3 antibodies of the present disclosure are capable of binding to the human, cynomolgus monkey, rat and mouse ANGPTL3 antigens with high affinity.

**Table 5. The results of the biacore assay for the anti-ANGPTL3 antibodies and the ANGPTL3 proteins**

| Antibody | Antigen | Binding constant (1/Ms) | Dissociation constant (1/s) | Affinity (M) |
|---|---|---|---|---|
| P8G | Human ANGPTL3 | 2.20E+06 | 3.33E-04 | 1.51E-10 |
| | Cynomolgus monkey ANGPTL3 | 8.37E+06 | 4.97E-04 | 5.93E-11 |
| | Mouse ANGPTL3 | 9.70E+05 | 3.45E-04 | 3.55E-10 |
| | Rat ANGPTL3 | 1.34E+06 | 3.35E-04 | 2.50E-10 |
| P8BG | Human ANGPTL3 | 6.14E+06 | 2.76E-04 | 4.50E-11 |
| | Cynomolgus monkey ANGPTL3 | 5.18E+06 | 3.98E-04 | 7.69E-11 |
| | Mouse ANGPTL3 | 1.46E+06 | 4.20E-04 | 2.88E-10 |
| | Rat ANGPTL3 | 1.21E+06 | 3.11E-04 | 2.57E-10 |

### Example 5: Pharmacodynamic Evaluation of Anti-ANGPTL3 Antibodies in Rats

### 5.1. Lipid lowering experiment of the antibody P8BG in rats

SD rats for experiments (4-week-old SD rats, SPF, about 100 g, male, purchased from Shanghai Slac Laboratory Animal Co., Ltd.; certification No. 20170005013017, SCXK (Shanghai) 2017-0005) were acclimatized to the laboratory conditions (under a 12/12 hour light/dark cycle, temperature of 23 ± 1 °C, humidity of 40-50%) for 1 week, and the animals were all given standard sterile mouse feed and *ad libitum* access to food and water. 7 days before the start of the experiment, after the animals were fasted for 4 h, blood was collected from the orbit and centrifuged at 3500 RPM for 15 min, and the serum was collected. The lipid concentration (triglycerides) in the serum was determined using the ADVIA 2400 chemical system (Siemens). The rats were divided into 6 groups of 6 by triglyceride level (non-relevant isotype human IgG protein (hereinafter hIgG) as a negative control group, evinacumab (for its sequence structure, see the sequence of evinacumab in WHO Drug Information, Vol. 29, No. 3, 2015) 3.5 mpk group, evinacumab 7 mpk group, P8BG 3.5 mpk group, P8BG 1.75 mpk group, and P8BG 7 mpk group) and subcutaneously injected once. On days 1, 5, 9, 13 and 16 after the injections, after these groups of rats were fasted for 4 h, blood was collected and the serum was separated. The lipid concentration (triglycerides) in the serum was determined using the ADVIA 2400 chemical system (Siemens). The experimental data were expressed as Mean ± standard deviation (Mean ± SEM) and plotted using Graphpad Prism5 software, and statistical analysis was performed using TTEST.

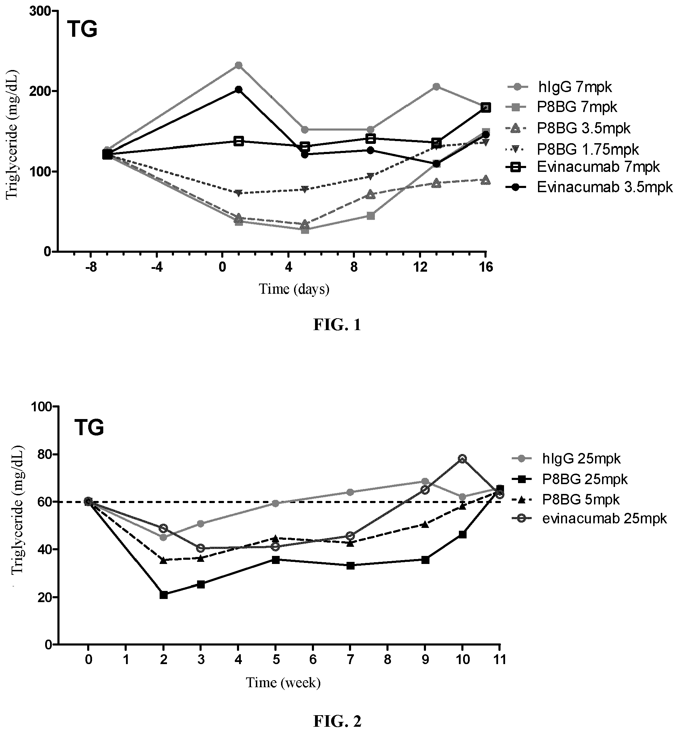

The experimental results are shown in FIG. 1 and Table 6. Compared with the negative control group, 1 day after the administration, the triglyceride level of each treatment group decreased, and statistical differences were observed in the groups except for the evinacumab 3.5 mpk group. During the experiment, the triglyceride level of the P8BG 7 mpk group decreased by a maximum of 83.7%, the triglyceride level of the P8BG 3.5 mpk group decreased by a maximum of 81.8%, and the triglyceride level of the P8BG 1.75 mpk group decreased by a maximum of 68.7%; 5 days after the administration, there were still statistical differences between the decreases in the triglyceride levels of the P8BG 3.5 mpk group, the P8BG 1.75 mpk group, and the P8BG 7 mpk group, while there was no statistical difference between the decreases in those of the evinacumab 3.5 mpk group and the evinacumab 7 mpk group; 9 days after the administration, there was still a statistical difference between the decreases in those of the P8BG 7 mpk group and the P8BG 3.5 mpk group. This indicates that in the same dose, P8BG has a better and more lasting triglyceride lowering effect than evinacumab. In addition, the body weight of the animals in each group was normal during the experiment.

**Table 6. The effect of the antibody P8BG on serum triglycerides in rats**

| Antibody | Evinacumab | | P8BG | | |
|---|---|---|---|---|---|
| Dose for subcutaneous administration | 7mpk | 3.5mpk | 7mpk | 3.5mpk | 1.75mpk |
| Initial TG (mg/dL) | 121.5±17.0 | 122.2±17.1 | 120.1±14.3 | 122.6±15.6 | 120.9±16.1 |
| Minimum TG level | 131.1±12.5 | 109.7±16.7 | 27.6±3.1 | 34.2±4.7 | 72.6±10.9 |
| Max Inh % | 40.7% | 13.1% | 83.7% | 81.8% | 68.7% |

| | | | | | |
|---|---|---|---|---|---|
| (Note: Max Inh% represents the maximum percent decrease in the lipid level relative to the TG of the negative control group; mpk represents mg/kg.) | | | | | |

### 5.2. Determination of in vivo effect of anti-ANGPTL3 antibodies on plasma lipid concentration in high-fat high-cholesterol fed c57b1/6 mice

c57bl/6 mice were kept in cages, 5 mice/cage (4-week-old c57bl/6 mice, SPF, about 16-18 g, male, purchased from Shanghai Cavens Laboratory Animal Co., Ltd.; certification No. 201913812, SCXK (Jiangsu) 2016-0010). The mice were acclimatized to the laboratory conditions (under a 12/12 hour light/dark cycle, temperature of 23 ± 1 °C, humidity of 40-50%) for 4 weeks, and the animals were all fed with common feed and given *ad libitum* access to food and water. 5 days after pre-blood collection, the common feed was changed to high-fat high-cholesterol feed (D12079B, purchased from Jiangsu Xietong Pharmaceutical Bio-engineering Co., Ltd.) for feeding the mice until the end of the experiment. 8 days before the start of the experiment, after the animals were fasted for 4 h, blood was collected from the orbit and centrifuged at 8000 RPM for 2 min, and the plasma was collected. The lipid concentration in the plasma was determined using the ADVIA 2400 chemical system (Siemens). The mice were divided into 4 groups of 11 by triglyceride level (hIgG negative control group, evinacumab 25 mpk group, P8BG 25 mpk group, and P8BG 5 mpk group) and subcutaneously injected once a week, and a total of 8 administrations were performed. At weeks 2, 3, 5, 7, 9, 10 and 11 after the start of the experiment, after the mice in each group were fasted for 4 h, blood was collected from the orbit, and the plasma was collected. The lipid concentrations (triglycerides (TG), total cholesterol (TC) and LDL cholesterol (LDL-C)) in the plasma were determined using the ADVIA 2400 chemical system (Siemens). The experimental data were expressed as Mean ± standard deviation (Mean ± SEM) and plotted using Graphpad Prism5 software, and statistical analysis was performed using TTEST.

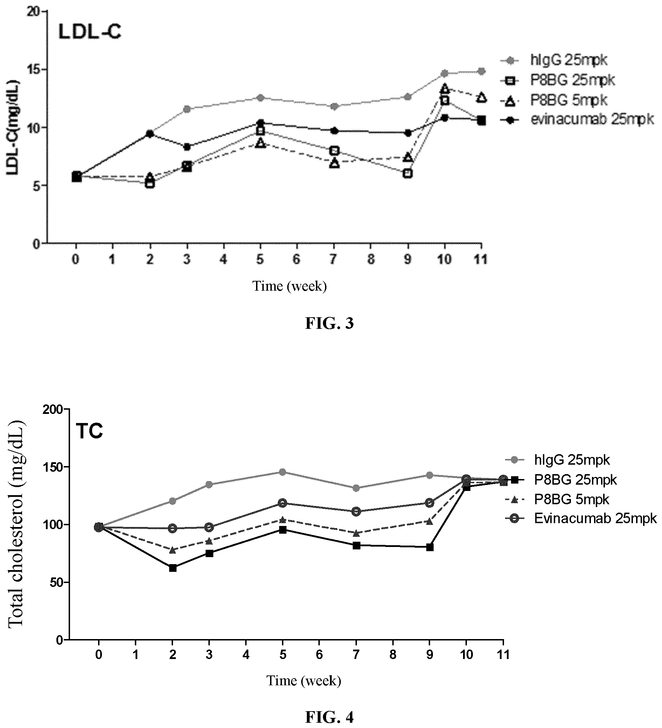

The results are shown in Tables 7-9 and FIGs. 2-4. Compared with the hIgG negative control group, the P8BG 25 mpk group showed significantly decreases in the TG, TC and LDL-C levels 2, 3, 5 and 7 weeks after the start of the experiment; the evinacumab 25 mpk group and the P8BG 5 mpk group showed decreases in the TG, TC and LDL-C levels 3, 5 and 7 weeks after the start of the experiment. 9 weeks after the start of the experiment, only two groups, the P8BG 25 mpk group and the P8BG 5 mpk group, showed significant decreases in the triglyceride level compared with the hIgG negative control group. Compared with the evinacumab 25 mpk group, the P8BG 25 mpk group showed significantly decreases in the triglyceride and total cholesterol levels at weeks 2, 3 and 9 of the experiment; compared with the evinacumab 25 mpk group, the P8BG 25 mpk group showed significant decreases in the LDL-C level at weeks 2 and 9 of the experiment. Throughout the entire experiment cycle from week 1 to week 11, the TG level of the evinacumab 25 mpk group decreased to a minimum of 40.5 ± 2.3-a 32.60% decrease compared with the initial TG; and the TG of the P8BG 25 mpk group decreased to a minimum of 21.1 ± 1.3-a 64.90% decrease compared with the initial TG. This indicates that the antibody P8BG has a better and more lasting lipid lowering effect than evinacumab.

**Table 7. The effect of the anti-ANGPTL3 antibodies on plasma TG in c57bl/6 mice**

| Antibody | Evinacumab | P8BG | | hIgG |
|---|---|---|---|---|
| Dose for weekly subcutaneous administration | 25mpk | 25mpk | 5mpk | 25mpk |
| Initial TG (mg/dL) | 60.1±3.2 | 60.1±4.1 | 60.1±3.8 | 60.0±6.2 |
| Minimum TG level | 40.5±2.3 | 21.1±1.3 | 35.5±1.7 | 45.1±7.1 |
| max Inh % | 32.6% | 64.9% | 40.9% | 24.8% |

| | | | | |
|---|---|---|---|---|
| (Note: max Inh% represents the maximum percent decrease in the lipid level relative to the initial TG) | | | | |

**Table 8. The effect of the anti-ANGPTL3 antibodies on plasma LDL in c57bl/6 mice**

| Antibody | Evinacumab | P8BG | |
|---|---|---|---|
| Dose for weekly subcutaneous administration | 25mpk | 25mpk | 5mpk |
| Max Inh % | 28.10% | 52.10% | 42.80% |

| | | | |
|---|---|---|---|
| (Note: Max Inh% represents the maximum percent decrease in the lipid level relative to the LDL of the negative control group) | | | |

**Table 9. The effect of the anti-ANGPTL3 antibodies on plasma TC in c57bl/6 mice**

| Antibody | Evinacumab | P8BG | |
|---|---|---|---|
| Dose for weekly subcutaneous administration | 25mpk | 25mpk | 5mpk |
| Max Inh % | 27.50% | 47.90% | 36.20% |

| | | | |
|---|---|---|---|
| (Note: Max Inh% represents the maximum percent decrease in the lipid level relative to the TC of the negative control group) | | | |

### 5.3. Determination of in vivo effect of anti-ANGPTL3 antibodies on plasma lipid concentration in APOE mice

APOE mice were kept in cages, 2 mice/cage (8-week-old APOE mice, SPF, about 22 g, male, purchased from Shanghai Cavens Laboratory Animal Co., Ltd.; certification No. 201917260, SCXK (Jiangsu) 2016-0010). The mice were acclimatized to the laboratory conditions (under a 12/12 hour light/dark cycle, temperature of 23 ± 1 °C, humidity of 40-50%) for 4 weeks, and the animals were all fed with high-fat feed (D12108C) and given *ad libitum* access to food and water. 7 days before the start of the experiment, after the animals were fasted for 4 h, blood was collected from the orbit and centrifuged at 8000 RPM for 2 min, and the plasma was collected. The lipid concentration in the plasma was determined using the ADVIA 2400 chemical system (Siemens). Mice were divided into 5 groups of 8 by triglyceride level (hIgG negative control group, evinacumab 10 mpk group, evinacumab 5 mpk group, P8BG 10 mpk group, and P8BG 5 mpk group) and subcutaneously injected once (see Table 6 for the dose for each group). On days (d) 1, 7 and 11 after the administration, after these groups of mice were fasted for 4 h, blood was collected from the orbit, and the plasma was collected. The lipid concentrations (triglycerides and HDL cholesterol) in the plasma were determined using the ADVIA 2400 chemical system (Siemens). The experimental data were expressed as Mean ± standard deviation (Mean ± SEM) and plotted using Graphpad Prism5 software, and statistical analysis was performed using TTEST.

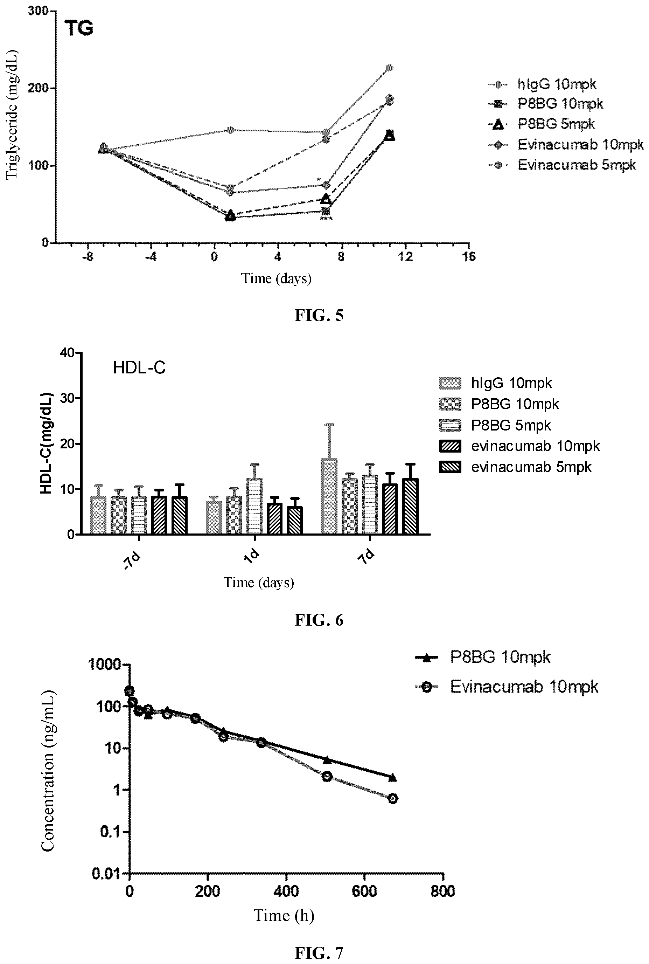

The experimental results are shown in FIGs. 5 and 6 and Table 10. Compared with the hIgG negative control group, 1 day after the administration, each treatment group showed a decrease in the triglyceride level. The triglyceride level of the evinacumab (10 mpk) group decreased to a minimum of 65.6 ± 5.4 mg/dL-a 46.2% decrease compared with the initial TG value; the triglyceride level of the evinacumab (5 mpk) group decreased to a minimum of 71.8 ± 3.3 mg/dL-a 42.1% decrease compared with the initial TG value; the triglyceride level of the P8BG (10 mpk) group decreased to a minimum of 33.3 ± 2.2 mg/dL-a 72.9% decrease compared with the initial TG value; the triglyceride level of the P8BG (5 mpk) group decreased to a minimum of 37.0 ± 1.7 mg/dL-a 70.2% decrease compared with the initial TG value. In addition, the experimental results show that 1 day after the administration, the plasma HDL-C level of the P8BG 10 mpk group increased, and the plasma HDL-C level of the evinacumab 10 mpk group decreased. However, in the lipid lowering experiment, the decrease in the HDL-C level is not normally desired when the lipid level decreased.

**Table 10. The effect of the anti-ANGPTL3 antibodies on plasma triglycerides in APOE mice**

| Antibody | Evinacumab | | P8BG | | hIgG |
|---|---|---|---|---|---|
| Dose for subcutaneous administration | 10 mpk | 5 mpk | 10 mpk | 5 mpk | 10 mpk |
| Initial TG (mg/dL) | 122.0±8.8 | 123.9±17.1 | 122.8±10.7 | 124.0±20.8 | 120.5±14.9 |
| Minimum TG level | 65.6±5.4 | 71.8±3.3 | 33.3±2.2 | 37.0±1.7 | 146.7±21.6 |
| max Inh % | 46.20% | 42.10% | 72.90% | 70.20% | -21.70% |

| | | | | | |
|---|---|---|---|---|---|
| (Note: max Inh% represents the maximum percent decrease in the lipid level relative to the initial TG) | | | | | |

### Example 6: Pharmacokinetic Evaluation of Anti-ANGPTL3 Antibodies

### 6.1. Pharmacokinetic experiment of anti-ANGPTL3 antibodies in mice

C57BL/6 mice weighing 18-24 g (purchased from Zhejiang Vital River Laboratory Animal Technology Co., Ltd.) were acclimatized to the laboratory conditions (under a 12/12 hour light/dark cycle, temperature of 16-26 °C, relative humidity of 40-70%) for no less than 3 days. During the acclimatization, the mice were given *ad libitum* access to feed and water. The day before the start of the experiment, the C57BL/6 mice were numbered and randomized into groups of 3. On the day of the experiment, these groups of mice were intravenously injected with the test drugs P8BG and evinacumab. The administration dose was 10 mg/kg, and the injection volume was 5 mL/kg.

Whole blood samples of 0.1 mL were collected from the administration groups before the administration and 5 min, 8 h, 1 d, 2 d, 4 d, 7 d, 10 d, 14 d, 21 d and 28 d after the administration, and no anticoagulant was added. The collected blood samples were left at 4 °C for 30 min and centrifuged at 1000 g for 15 min. The supernatants (serum) were transferred to EP tubes and stored at -80 °C.

The antibody concentration in serum was determined by ELISA, and the pharmacokinetic parameters were calculated for the test drugs by Winnolin software. The main pharmacokinetic results obtained are shown in Table 11 and FIG. 7. The experimental results show that the antibody P8BG of the present disclosure has a higher half-life in mice than the positive control evinacumab.

**Table 11. The pharmacokinetics of the anti-ANGPTL3 antibodies in mice**

| Antibody | P8BG | Evinacumab |
|---|---|---|
| Route of administration | i.v. | i.v. |
| Dose (mg/kg) | 10 | 10 |
| t_{1/2} (h) | 131.4±18.8 | 71.1±6.1 |
| t_{1/2} (d) | 5.47±0.78 | 2.96±0.25 |
| AUC0-∞ (h*ug/mL) | 23272±2887 | 16904±4481 |

| | | |
|---|---|---|
| (Note: AUC0-∞: area under the plasma drug concentration-time curve; t_{1/2} (h): half-life (in hours); t_{1/2} (d): half-life (in days); i.v.: intravenous injection.) | | |

### 6.2. Pharmacokinetic evaluation of anti-ANGPTL3 antibodies in cynomolgus monkeys

Cynomolgus monkeys weighing 3-4 kg (purchased from Guangdong Qianyan Biological Science and Technology Co., Ltd.) were used. The animals were subjected to a quarantine of at least 14 days and to adaptive feeding before being used in the experiment. The animals were kept in stainless steel cages, with no more than 2 animals per cage. The room temperature of the animal room was controlled at 18 °C-26 °C, and the relative humidity was at 40%-70%. The animals were kept under a 12-hour light-dark cycle. The animals were given *ad libitum* access to water during the experiment. 6 cynomolgus monkeys were randomized into 2 groups of 3 according to biochemical indicators. These groups were subcutaneously injected with the test drugs P8BG and evinacumab. The administration dose was 10 mg/kg, and the injection volume was 2 mL/kg.

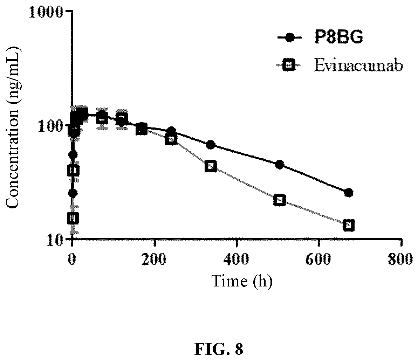

After the grouping, the animals were fasted overnight. Blood samples of about 2 mL were collected from a vein of a lower limb before the administration and 1 hour (h), 2 h, 4 h, 8 h, 12 h, 1 d, 3 d, 5 d, 7 d, 10 d, 14 d, 21 d, 28 d, 35 d, 42 d, 49 d and 56 d after the start of the administration. The blood samples were placed in blood collection tubes containing separation gel, left to stand at room temperature for 30 min, and centrifuged at 1000 g for 15 min. The supernatants were collected, aliquoted into 2 clean EP tubes, and temporarily stored at -70 °C (the above procedure was completed within 2 h of collecting blood). The antibody concentration in serum was determined by ELISA, and the pharmacokinetic parameters were calculated for the test drugs by Winnolin software. The main pharmacokinetic results obtained are shown in Table 12 and FIG. 8. The experimental results show that the antibody P8BG of the present disclosure has a higher half-life in cynomolgus monkeys than the positive control evinacumab.

**Table 12. The pharmacokinetics of the anti-ANGPTL3 antibodies in cynomolgus monkeys**

| | P8BG | Evinacumab |
|---|---|---|
| Dose (mg/kg) | 10 | 10 |
| Route of administration | sc. | sc. |
| t_{1/2} (h) | 324.7±115.3 | 157.7±28.6 |
| t_{1/2} (d) | 13.5±4.8 | 6.6±1.2 |
| AUC 0-∞ (ug/mL*h) | 44243±7404 | 38027±252 |

| | | |
|---|---|---|
| (Note: AUC 0-∞: area under the plasma drug concentration-time curve; t_{1/2} (h): half-life (in hours); t_{1/2} (d): half-life (in days); sc.: subcutaneous injection.) | | |

### Example 7: Preparation Process for Exemplary Pharmaceutical Composition (Formulation) of Antibody

Step 1: A stock solution containing an anti-ANGPTL3 antibody was prepared with the anti-ANGPTL3 antibody and a stabilizer. After filtration, an in-process control sample was taken and analyzed. The analysis showed that the stock solution was germ-free. The stock solution was passed through a 0.22 µm PVDF filter, and the filtrate was collected.

Step 2: The filling amount was adjusted, and filling (the filling amount and the packaging material were to be determined) and plugging were carried out. In-process control samples were taken at the beginning of the filling, during the filling and at the end of the filling and detected for differences in filling amount.

Step 3: The capping machine was started, aluminum caps were put on, and capping was carried out.

Step 4: Visual inspection was performed to confirm that the products have no defects, such as inaccurate filling amount. Vial labels were printed and put on the vials; carton labels were printed, cartons were folded, packing was performed, and the carton labels were put on the cartons.

The anti-ANGPTL3 antibody used in Examples 7-13 was the antibody P8BG, of which the heavy chain sequence is set forth in SEQ ID NO: 21 and the light chain sequence is set forth in SEQ ID NO: 22.

### Example 8: Screening for pH Values for Anti-ANGPTL3 Antibody Formulations

The following buffers were formulated, and antibody formulations with an anti-ANGPTL3 antibody concentration of 1.0 mg/mL were prepared. By measuring the melting temperature (Tm) and the thermal decomposition onset temperature (Tonset) of samples, the thermal stability of the anti-ANGPTL3 antibody under different pH conditions was investigated. By measuring the aggregation temperature (Tagg) and the particle size (Radius) of samples, the colloidal stability of the anti-ANGPTL3 antibody under different pH conditions was investigated.
1) 10 mM sodium dihydrogen phosphate-disodium hydrogen phosphate, pH 4.2
2) 10 mM sodium dihydrogen phosphate-disodium hydrogen phosphate, pH 4.6
3) 10 mM sodium dihydrogen phosphate-disodium hydrogen phosphate, pH 5.0
4) 10 mM sodium dihydrogen phosphate-disodium hydrogen phosphate, pH 5.4
5) 10 mM sodium dihydrogen phosphate-disodium hydrogen phosphate, pH 5.8
6) 10 mM sodium dihydrogen phosphate-disodium hydrogen phosphate, pH 6.2
7) 10 mM sodium dihydrogen phosphate-disodium hydrogen phosphate, pH 6.6
8) 10 mM sodium dihydrogen phosphate-disodium hydrogen phosphate, pH 7.0
9) 10 mM sodium dihydrogen phosphate-disodium hydrogen phosphate, pH 7.4
10) 10 mM sodium dihydrogen phosphate-disodium hydrogen phosphate, pH 7.8
11) 10 mM sodium dihydrogen phosphate-disodium hydrogen phosphate, pH 8.2
12) 10 mM sodium dihydrogen phosphate-disodium hydrogen phosphate, pH 8.6

**Table 13. The results of the anti-ANGPTL3 antibody pH value screening**

| No. | Calculated pH | Tm1 (°C) | Tm2 (°C) | Tonset (°C) | Tagg 266 (°C) | Tagg 473 (°C) | Particle size (nm) | %PD |
|---|---|---|---|---|---|---|---|---|
| 1) | 4.2 | 51.8 | 67.5 | 59.6 | 68.6 | 68.9 | 6.0 | 12.7 |
| 2) | 4.6 | 54.4 | 68.9 | 61.8 | 68.2 | 68.5 | 5.8 | 11.4 |
| 3) | 5.0 | 55.4 | 68.4 | 64.1 | 67.6 | 68.0 | 6.1 | 17.7 |
| 4) | 5.4 | 58.0 | 67.8 | 62.9 | 66.5 | 67.2 | 6.0 | 16.0 |
| 5) | 5.8 | 59.1 | 67.3 | 60.9 | 65.5 | 66.4 | 5.4 | 2.7 |
| 6) | 6.2 | 60.1 | 66.3 | 61.6 | 63.5 | 65.3 | 5.4 | 0.1 |
| 7) | 6.6 | 65.3 | N/A | 59.7 | 64.8 | 65.3 | 5.4 | 3.1 |
| 8) | 7.0 | 63.9 | N/A | 58.6 | 64.9 | 65.3 | 6.1 | 21.5 |
| 9) | 7.4 | 62.8 | N/A | 58.4 | 64.2 | 64.7 | 6.2 | 24.1 |
| 10) | 7.8 | 62.3 | N/A | 57.4 | 62.9 | 62.9 | 6.1 | 20.6 |
| 11) | 8.2 | 62.0 | N/A | 57.5 | 62.5 | 63.5 | 6.2 | 22.4 |
| 12) | 8.6 | 61.8 | N/A | 57.5 | 61.0 | N/A | 6.5 | 27.9 |

| | | | | | | | | |
|---|---|---|---|---|---|---|---|---|
| (Note: %PD represents dispersion coefficient, and the smaller the value, the more uniform the particle size distribution; N/A represents not applicable) | | | | | | | | |

The results show that: at the pH of 5.0 ± 0.4, the Tagg and Tonset values of the anti-ANGPTL3 antibody were relatively high; at the pH of 6.2 ± 0.4, the particle sizes were relatively small, suggesting that the distributions were more uniform; the relatively high Tm1 values indicate that the antibody has relatively good thermal stability and colloidal stability at the pH of 5.0 ± 0.4 and 6.2 ± 0.4.

### Example 9: Screening for and Confirmation of Buffer Systems for Anti-ANGPTL3 Antibody Formulations

### 9.1. Screening of pH 6.0 buffer systems

The 10 mM sodium dihydrogen phosphate-disodium hydrogen phosphate pH 6.0 and 10 mM histidine-histidine hydrochloride pH 6.0 buffer systems were selected to prepare formulations containing 50 mg/mL anti-ANGPTL3 antibody, and samples were taken for high-temperature (40 °C) and room-temperature (25 °C) stability studies:
1) 10 mM sodium dihydrogen phosphate-disodium hydrogen phosphate pH 6.0
2) 10 mM histidine-histidine hydrochloride pH 6.0

**Table 14. The stability results 1 of the anti-ANGPTL3 antibody buffer system (pH 6.0) screening**

| Buffer system | Tm1 (°C) | Tm2 (°C) | Tonset (°C) | Tagg 266 (°C) | Tagg 473 (°C) |
|---|---|---|---|---|---|
| 10 mM sodium dihydrogen phosphate-disodium hydrogen phosphate pH 6.0 | 57.5 | 67.2 | 58.4 | 64.1 | 65.4 |
| 10 mM histidine-histidine hydrochloride pH 6.0 | 54.4 | 69.0 | 58.3 | 67.9 | 67.6 |

| | | | | | |
|---|---|---|---|---|---|
| (Note: This table shows the time-0 stability results of the anti-ANGPTL3 antibody buffer system (pH 6.0) screening) | | | | | |

**Table 15. The stability results 2 of the anti-ANGPTL3 antibody buffer system (pH 6.0) screening**

| Buffer system | Investigation conditions | Appearance | Particle size (nm) | %PD |
|---|---|---|---|---|
| 10 mM sodium dihydrogen | Time 0 | Colorless, significantly opalescent, a large number of white particles | 7.1 | 4.3 |
| phosphate-disodium hydrogen phosphate pH 6.0 | 25°C-1W | Colorless, significantly opalescent, a large number of white particles | 7.1 | 6.3 |
| | 25°C-2W | Colorless, significantly opalescent, a large amount of white fiber | 7.1 | 5.2 |
| | 25°C-4W | Colorless, significantly opalescent, a large amount of white fiber | 7.2 | 6.3 |
| | 40°C-1W | Colorless, significantly opalescent, a large amount of white fiber | 7.3 | 8.8 |
| | 40°C-2W | Colorless, significantly opalescent, a large amount of white fiber | 7.5 | 13.2 |
| | 40°C-4W | Colorless, significantly opalescent, a large amount of white fiber | 7.9 | 17.5 |
| 10 mM histidine-histidine hydrochlorid e pH 6.0 | Time 0 | Colorless, significantly opalescent, a large number of white particles | 6.3 | 3.1 |
| | 25°C-1W | Colorless, significantly opalescent, a large number of white particles | 6.8 | 4.9 |
| | 25°C-2W | Colorless, significantly opalescent, a large number of white particles | 6.7 | 6.3 |
| | 25°C-4W | Colorless, significantly opalescent, a large amount of white fiber | 6.7 | 4.4 |
| | 40°C-1W | Colorless, significantly opalescent, a large number of white particles | 6.7 | 5.1 |
| | 40°C-2W | Colorless, significantly opalescent, a large amount of white fiber | 6.8 | 5.7 |
| | 40°C-4W | Colorless, significantly opalescent, a large amount of white fiber | 7.0 | 10.6 |

| | | | | |
|---|---|---|---|---|
| (Note: 1W represents 1 week; particle size reflects colloidal stability; %PD represents dispersion coefficient) | | | | |

**Table 16. The stability results 3 of the anti-ANGPTL3 antibody buffer system (pH 6.0) screening**

| Buffer system | Investigation conditions | SEC | | iCIEF | | |
|---|---|---|---|---|---|---|
| | | Aggregate% | Monomer% | Acidic peak% | Main peak% | Basic peak% |
| 10 mM | Time 0 | 5.1 | 94.9 | 15.3 | 43.4 | 41.3 |
| sodium dihydrogen phosphate-disodium hydrogen phosphate pH 6.0 | 25°C-1W | 5.1 | 94.9 | 15.5 | 44.2 | 40.4 |
| | 25°C-2W | 5.1 | 94.9 | 16.6 | 43.2 | 40.3 |
| | 25°C-4W | 5.2 | 94.8 | 17.1 | 45.0 | 38.0 |
| | 40°C-1W | 5.6 | 94.4 | 16.9 | 44.7 | 38.4 |
| | 40°C-2W | 6.0 | 93.5 | 20.1 | 42.8 | 37.1 |
| | 40°C-4W | 7.1 | 92.2 | 27.6 | 35.5 | 36.9 |
| 10 mM histidine-histidine hydrochloride pH 6.0 | Time 0 | 4.6 | 95.4 | 14.9 | 43.9 | 41.2 |
| | 25°C-1W | 4.5 | 95.5 | 15.9 | 44.8 | 39.4 |
| | 25°C-2W | 4.5 | 95.5 | 16.3 | 45.2 | 38.6 |
| | 25°C-4W | 4.6 | 95.4 | 16.6 | 48.3 | 35.1 |
| | 40°C-1W | 5.0 | 95.0 | 16.7 | 44.9 | 38.4 |
| | 40°C-2W | 5.3 | 94.2 | 18.5 | 43.7 | 37.8 |
| | 40°C-4W | 6.1 | 93.2 | 25.7 | 35.6 | 38.7 |

| | | | | | | |
|---|---|---|---|---|---|---|
| (Note: 1W represents 1 week) | | | | | | |

The results show that: under the same investigation conditions, the anti-ANGPTL3 antibody has a higher Tagg value, a smaller particle size and a lower aggregate content in the 10 mM histidine-histidine hydrochloride pH 6.0 buffer system than in the 10 mM sodium dihydrogen phosphate-disodium hydrogen phosphate pH 6.0 buffer system, indicating that the anti-ANGPTL3 antibody has better stability in the 10 mM histidine-histidine hydrochloride pH 6.0 buffer system.

### 9.2. Screening of pH 6.6 buffer systems

The 10 mM sodium dihydrogen phosphate-disodium hydrogen phosphate pH 6.6 and 10 mM histidine-histidine hydrochloride pH 6.6 buffer systems were selected to prepare formulations containing 50 mg/mL anti-ANGPTL3 antibody, and samples were taken for high-temperature (40 °C) and room-temperature (25 °C) stability studies:
1) 10 mM sodium dihydrogen phosphate-disodium hydrogen phosphate pH 6.6
2) 10 mM histidine-histidine hydrochloride pH 6.6

**Table 17. The stability results 1 of the anti-ANGPTL3 antibody buffer system (pH 6.6) screening**

| Buffer system | Tm1 (°C) | Tm2 (°C) | Tonset (°C) | Tagg 266 (°C) | Tagg 473 (°C) |
|---|---|---|---|---|---|
| 10 mM sodium dihydrogen phosphate-disodium hydrogen phosphate pH 6.6 | 59.5 | 66.2 | 59.2 | 63.2 | 64.7 |
| 10 mM histidine-histidine hydrochloride pH 6.6 | 59.0 | 67.0 | 55.8 | 65.5 | 65.3 |

| | | | | | |
|---|---|---|---|---|---|
| (Note: This table shows the time-0 stability results of the anti-ANGPTL3 antibody buffer system (pH 6.6) screening) | | | | | |

**Table 18. The stability results 2 of the anti-ANGPTL3 antibody buffer system (pH 6.6) screening**

| Buffer system | Investigation conditions | Appearance | Particle size (nm) | %PD |
|---|---|---|---|---|
| 10 mM sodium dihydrogen phosphate-disodium hydrogen phosphate pH 6.6 | Time 0 | Colorless, significantly opalescent, a large number of white particles | 6.8 | 6.8 |
| | 25°C-1W | Colorless, significantly opalescent, a large number of white particles | 6.8 | 5.2 |
| | 25°C-2W | Colorless, significantly opalescent, a large amount of white fiber | 6.7 | 7.1 |
| | 25°C-4W | Colorless, significantly opalescent, a large amount of white fiber | 6.8 | 6.1 |
| | 40°C-1W | Colorless, significantly opalescent, a large amount of white fiber | 7.0 | 10.0 |
| | 40°C-2W | Colorless, significantly opalescent, a large amount of white fiber | 7.1 | 13.6 |
| | 40°C-4W | Colorless, significantly opalescent, a large amount of white fiber | 7.7 | 20.7 |
| 10 mM histidine-histidine hydrochloride pH 6.6 | Time 0 | Colorless, significantly opalescent, a large number of white particles | 7.8 | 3.4 |
| | 25°C-1W | Colorless, significantly opalescent, a large number of white particles | 8.0 | 4.1 |
| | 25°C-2W | Colorless, significantly opalescent, a large number of white particles | 7.8 | 3.8 |
| | 25°C-4W | Colorless, significantly opalescent, a large number of white particles | 7.4 | 10.8 |
| | 40°C-1W | Colorless, significantly opalescent, a large amount of white fiber | 8.2 | 7.7 |
| | 40°C-2W | Colorless, significantly opalescent, a large amount of white fiber | 8.1 | 6.5 |
| | 40°C-4W | Colorless, significantly opalescent, a large amount of white fiber | 6.8 | 18.5 |

| | | | | |
|---|---|---|---|---|
| (Note: 1W represents 1 week; particle size reflects colloidal stability; %PD represents dispersion coefficient) | | | | |

**Table 19. The stability results 3 of the anti-ANGPTL3 antibody buffer system (pH 6.6) screening**

| Buffer system | Investigation conditions | SEC | | iCIEF | | |
|---|---|---|---|---|---|---|
| | | Aggregate% | Monomer% | Acidic peak% | Main peak% | Basic peak% |
| 10 mM sodium dihydrogen phosphate-disodium hydrogen phosphate pH 6.6 | Time 0 | 4.7 | 95.3 | 15.5 | 43.9 | 40.6 |
| | 25°C-1W | 4.7 | 95.3 | 16.6 | 43.5 | 39.9 |
| | 25°C-2W | 4.6 | 95.4 | 16.5 | 43.4 | 40.1 |
| | 25°C-4W | 4.8 | 95.2 | 17.1 | 44.2 | 38.7 |
| | 40°C-1W | 5.1 | 94.8 | 19.2 | 43.5 | 37.4 |
| | 40°C-2W | 5.6 | 94.3 | 22.2 | 43.1 | 34.7 |
| | 40°C-4W | 6.6 | 92.8 | 32.5 | 34.1 | 33.4 |
| 10 mM histidine-histidine hydrochloride pH 6.6 | Time 0 | 4.5 | 95.5 | 15.2 | 44.3 | 40.5 |
| | 25°C-1W | 4.5 | 95.5 | 15.4 | 44.6 | 40.0 |
| | 25°C-2W | 4.4 | 95.6 | 16.0 | 43.7 | 40.3 |
| | 25°C-4W | 4.5 | 95.4 | 16.2 | 44.8 | 38.9 |
| | 40°C-1W | 4.8 | 95.1 | 17.4 | 43.0 | 39.6 |
| | 40°C-2W | 5.1 | 94.5 | 20.1 | 42.9 | 37.0 |
| | 40°C-4W | 5.8 | 93.5 | 30.3 | 38.0 | 31.6 |

| | | | | | | |
|---|---|---|---|---|---|---|
| (Note: 1W represents 1 week) | | | | | | |

The results show that: under the same investigation conditions, the anti-ANGPTL3 antibody has a lower Tonset value, a higher Tagg value, and a larger particle size, but has a relatively lower aggregate content, a relatively higher monomer content, and a relatively higher main peak content in the 10 mM histidine-histidine hydrochloride pH 6.6 buffer system than in the 10 mM sodium dihydrogen phosphate-disodium hydrogen phosphate pH 6.6 buffer system. Overall, the anti-ANGPTL3 antibody is considered to have better stability in the 10 mM histidine-histidine hydrochloride pH 6.6 buffer system.

### 9.3. Screening of pH 4.6 buffer systems

The 10 mM succinic acid-sodium succinate pH 4.6 and 10 mM acetic acid-sodium acetate pH 4.6 buffer systems were selected to prepare antibody formulations containing 50 mg/mL protein, and samples were taken for high-temperature (40 °C) and room-temperature (25 °C) stability studies:
1) 10 mM succinic acid-sodium succinate pH 4.6
2) 10 mM acetic acid-sodium acetate pH 4.6

**Table 20. The stability results 1 of the anti-ANGPTL3 antibody buffer system (pH 4.6) screening**

| Buffer system | Tm1 (°C) | Tm2 (°C) | Tonset (°C) | Tagg 266 (°C) | Tagg 473 (°C) |
|---|---|---|---|---|---|
| 10 mM succinic acid-sodium succinate pH 4.6 | 63.3 | 72.2 | 61.4 | N/A | 68.2 |
| 10 mM acetic acid-sodium acetate pH 4.6 | 60.5 | 71.4 | 65.1 | N/A | 67.9 |

| | | | | | |
|---|---|---|---|---|---|
| (Note: This table shows the time-0 stability results of the anti-ANGPTL3 antibody buffer system (pH 4.6) screening; N/A represents not applicable) | | | | | |

**Table 21. The stability results 2 of the anti-ANGPTL3 antibody buffer system (pH 4.6) screening**

| Buffer system | Investigation conditions | Appearance | Particle size (nm) | %PD |
|---|---|---|---|---|
| 10 mM succinic acid-sodium succinate pH 4.6 | Time 0 | Colorless, clear, a large number of white particles | 3.1 | 34.9 |
| | 25°C-1W | Colorless, clear, a large number of white particles | 3.4 | 48.7 |
| | 25°C-2W | Colorless, clear, a small number of white particles | 3.2 | 26.2 |
| | 25°C-4W | Colorless, clear, a tiny number of white particles | 3.3 | 35.6 |
| | 40°C-1W | Colorless, clear, a small number of white particles | 4.1 | N/A |
| | 40°C-2W | Colorless, slightly opalescent, a small number of white particles | 4.5 | N/A |
| | 40°C-4W | Colorless, clear, a tiny number of white particles | 5.1 | N/A |
| 10 mM acetic acid-sodium acetate pH 4.6 | Time 0 | Colorless, clear, a large number of white particles | 2.7 | 23.1 |
| | 25°C-1W | Colorless, clear, a large number of white particles | 2.7 | 21.4 |
| | 25°C-2W | Colorless, clear, a negligible amount of white fiber | 2.7 | 17.7 |
| | 25°C-4W | Colorless, clear, no visible protein particles | 2.7 | 24.3 |
| | 40°C-1W | Colorless, clear, a tiny number of white particles | 3.1 | 44.1 |
| | 40°C-2W | Colorless, clear, a small number of white particles | 3.3 | 51.1 |
| | 40°C-4W | Colorless, clear, a tiny number of white particles | 4.0 | N/A |

| | | | | |
|---|---|---|---|---|
| (Note: 1W represents 1 week; particle size reflects colloidal stability; %PD represents dispersion coefficient; N/A represents not applicable) | | | | |

**Table 22. The stability results 3 of the anti-ANGPTL3 antibody buffer system (pH 4.6) screening**

| Buffer system | Investigation conditions | SEC | | iCIEF | | |
|---|---|---|---|---|---|---|
| | | Aggregate% | Monomer% | Acidic peak% | Main peak% | Basic peak% |
| 10 mM succinic acid-sodium succinate pH 4.6 | Time 0 | 5.0 | 95.0 | 15.4 | 42.7 | 41.9 |
| | 25°C-1W | 5.1 | 94.9 | 16.1 | 43.8 | 40.2 |
| | 25°C-2W | 5.0 | 95.0 | 16.0 | 43.7 | 40.3 |
| | 25°C-4W | 5.2 | 94.7 | 17.3 | 44.1 | 38.6 |
| | 40°C-1W | 19.9 | 79.1 | 14.4 | 35.0 | 50.6 |
| | 40°C-2W | 29.1 | 68.9 | 13.6 | 29.6 | 56.8 |
| | 40°C-4W | 40.7 | 55.1 | 13.2 | 19.6 | 67.3 |
| 10 mM acetic acid-sodium acetate pH 4.6 | Time 0 | 4.9 | 95.1 | 15.5 | 43.6 | 40.9 |
| | 25°C-1W | 5.0 | 95.0 | 16.3 | 46.3 | 37.5 |
| | 25°C-2W | 5.0 | 95.0 | 17.1 | 47.0 | 35.9 |
| | 25°C-4W | 5.1 | 94.8 | 18.7 | 49.6 | 31.7 |
| | 40°C-1W | 17.4 | 81.3 | 12.2 | 35.0 | 52.9 |
| | 40°C-2W | 24.9 | 72.6 | 13.5 | 25.3 | 61.2 |
| | 40°C-4W | 35.6 | 59.4 | 11.1 | 18.3 | 70.5 |

| | | | | | | |
|---|---|---|---|---|---|---|
| (Note: 1W represents 1 week) | | | | | | |

The results show that: under the same investigation conditions, the anti-ANGPTL3 antibody has a lower Tm₁ value, but has a higher Tonset value, a smaller particle size, and a lower aggregate content in the 10 mM acetic acid-sodium acetate pH 4.6 buffer system than in the 10 mM succinic acid-sodium succinate pH 4.6 buffer system, indicating that the anti-ANGPTL3 antibody has better stability in the 10 mM acetic acid-sodium acetate pH 4.6 buffer system.

### 9.4. Screening of pH 5.0 buffer systems

The 10 mM succinic acid-sodium succinate pH 5.0 and 10 mM acetic acid-sodium acetate pH 5.0 buffer systems were selected to prepare formulations containing 50 mg/mL anti-ANGPTL3 antibody, and samples were taken for high-temperature (40 °C) and room-temperature (25 °C) stability studies:
1) 10 mM succinic acid-sodium succinate pH 5.0
2) 10 mM acetic acid-sodium acetate pH 5.0

**Table 23. The stability results 1 of the anti-ANGPTL3 antibody buffer system (pH 5.0) screening**

| Buffer system | Tm1 (°C) | Tm2 (°C) | Tonset (°C) | Tagg 266 (°C) | Tagg 473 (°C) |
|---|---|---|---|---|---|
| 10 mM succinic acid-sodium succinate pH 5.0 | 48.7 | 69.6 | 49.7 | N/A | 67.7 |
| 10 mM acetic acid-sodium acetate pH 5.0 | 51.8 | 71.2 | 60.6 | N/A | 68.0 |

| | | | | | |
|---|---|---|---|---|---|
| (Note: This table shows the time-0 stability results of the buffer system (pH 5.0) screening; N/A represents not applicable) | | | | | |

**Table 24. The stability results 2 of the anti-ANGPTL3 antibody buffer system (pH 5.0) screening**

| Buffer system | Investigation conditions | Appearance | Particle size (nm) | %PD |
|---|---|---|---|---|
| 10 mM succinic acid-sodium succinate pH 5.0 | Time 0 | Colorless, slightly opalescent, a large number of white particles | 5.4 | 16.8 |
| | 25°C -1W | Colorless, slightly opalescent, a large number of white particles | 5.3 | 18.9 |
| | 25°C -2W | Colorless, slightly opalescent, a large amount of white fiber | 5.3 | 18.0 |
| | 25°C -4W | Colorless, slightly opalescent, a large amount of white fiber | 5.5 | 27.8 |
| | 40°C -1W | Colorless, slightly opalescent, a large number of white particles | 5.9 | 33.6 |
| | 40°C -2W | Colorless, slightly opalescent, a large amount of white fiber | 6.1 | 35.9 |
| | 40°C -4W | Colorless, slightly opalescent, a large amount of white fiber | 7.0 | 40.9 |
| 10 mM acetic acid-sodium acetate pH 5.0 | Time 0 | Colorless, slightly opalescent, a large number of white particles | 3.8 | 22.8 |
| | 25°C -1W | Colorless, slightly opalescent, a large number of white particles | 3.9 | 25.8 |
| | 25°C -2W | Colorless, slightly opalescent, a large number of white particles | 3.9 | 24.0 |
| | 25°C -4W | Colorless, clear, a small number of white particles | 4.0 | 29.2 |
| | 40°C -1W | Colorless, slightly opalescent, a large number of white particles | 4.1 | 36.0 |
| | 40°C -2W | Colorless, slightly opalescent, a large number of white particles | 4.3 | 50.2 |
| | 40°C -4W | Colorless, clear, a small number of white particles | 4.6 | 53.9 |

| | | | | |
|---|---|---|---|---|
| (Note: 1W represents 1 week; particle size reflects colloidal stability; %PD represents dispersion coefficient) | | | | |

**Table 25. The stability results 3 of the anti-ANGPTL3 antibody buffer system (pH 5.0) screening**

| Buffer system | Investigation conditions | SEC | | iCIEF | | |
|---|---|---|---|---|---|---|
| | | Aggregate% | Monomer% | Acidic peak% | Main peak% | Basic peak% |
| 10 mM succinic acid-sodium succinate pH 5.0 | Time 0 | 4.8 | 95.2 | 15.6 | 43.0 | 41.5 |
| | 25°C -1W | 4.8 | 95.2 | 15.6 | 44.4 | 39.9 |
| | 25°C -2W | 4.8 | 95.2 | 16.1 | 44.6 | 39.3 |
| | 25°C -4W | 4.9 | 95.0 | 17.5 | 44.1 | 38.4 |
| | 40°C -1W | 9.9 | 89.6 | 16.4 | 40.6 | 43.0 |
| | 40°C -2W | 13.3 | 86.0 | 16.0 | 36.6 | 47.4 |
| | 40°C -4W | 21.2 | 77.3 | 21.8 | 27.3 | 50.8 |
| 10 mM acetic acid-sodium acetate pH 5.0 | Time 0 | 4.7 | 95.3 | 15.2 | 43.9 | 41.0 |
| | 25°C -1W | 4.7 | 95.3 | 16.6 | 47.3 | 36.2 |
| | 25°C -2W | 4.7 | 95.3 | 18.0 | 48.4 | 33.6 |
| | 25°C -4W | 4.8 | 95.2 | 19.5 | 54.0 | 26.5 |
| | 40°C -1W | 7.4 | 92.1 | 15.2 | 42.4 | 42.4 |
| | 40°C -2W | 9.4 | 89.8 | 14.9 | 41.3 | 43.9 |
| | 40°C -4W | 14.1 | 84.2 | 20.3 | 30.1 | 49.6 |

| | | | | | | |
|---|---|---|---|---|---|---|
| (Note: 1W represents 1 week) | | | | | | |

The results show that: under the same investigation conditions, the anti-ANGPTL3 antibody has a higher Tm₁ value, a higher Tonset value, a higher Tagg value, a smaller particle size, a lower aggregate content, a higher monomer content, and a higher main peak content in the 10 mM acetic acid-sodium acetate pH 5.0 buffer system than in the 10 mM succinic acid-sodium succinate pH 5.0 buffer system, indicating that the anti-ANGPTL3 antibody has better stability in the 10 mM acetic acid-sodium acetate pH 5.0 buffer system.

The results of the anti-ANGPTL3 antibody buffer system screening show that: the anti-ANGPTL3 antibody has a better appearance, a relatively high Tm₁ value, a relatively high Tagg value, and a relatively high Tonset value in the 10 mM succinic acid-sodium succinate pH 4.6, 10 mM acetic acid-sodium acetate pH 4.6 and 10 mM acetic acid-sodium acetate pH 5.0 buffer systems, but at the high temperature of 40 °C, the aggregate content significantly increased; the anti-ANGPTL3 antibody has a relatively bad appearance, a relatively high Tm₁ value, a relatively high Tagg value, and a relatively high Tonset value in the 10 mM histidine-histidine hydrochloride pH 6.0 buffer system, and under high-temperature conditions, there was no significant increase in the aggregate content. Given that the sample used in the buffer system screening was a mixed sample, a monoclonal antibody sample was used to perform buffer system confirmation to further confirm the final pH and buffer system.

### 9.5. Confirmation of buffer systems for anti-ANGPTL3 antibody formulations

The 10 mM histidine-histidine hydrochloride pH 6.0, 10 mM succinic acid-sodium succinate pH 4.6, 10 mM acetic acid-sodium acetate pH 4.6 and 10 mM acetic acid-sodium acetate pH 5.0 buffer systems were selected to prepare formulations containing 50 mg/mL anti-ANGPTL3 antibody, and samples were taken for high-temperature (60 °C and 40 °C) stability studies:
1) 10 mM histidine-histidine hydrochloride, pH 6.0;
2) 10 mM succinic acid-sodium succinate, pH 4.6;
3) 10 mM acetic acid-sodium acetate, pH 4.6; and
4) 10 mM acetic acid-sodium acetate, pH 5.0.

**Table 26. The results 1 of the anti-ANGPTL3 antibody formulation buffer system confirmation**

| Buffer system | Investigation conditions | Appearance | Particle size (nm) | %PD |
|---|---|---|---|---|
| 10 mM histidine-histidine hydrochloride pH 6.0 | Time 0 | Colorless, significantly opalescent, a large number of white particles | 9.0 | Multiple peaks |
| | 60°C-3h | Colorless, significantly opalescent, no visible particles | Imcompleted | |
| | 40°C-4d | Colorless, significantly opalescent, a small number of white particles | NT | NT |
| | 40°C-7d | Colorless, significantly opalescent, a large number of white particles | 7.7 | 31.7 |
| 10 mM succinic acid-sodium succinate pH 4.6 | Time 0 | Colorless, slightly opalescent, a large number of white particles | 7.2 | Multiple peaks |
| | 60°C-3h | Colorless, slightly opalescent, no visible particles | 7.2 | 57.0 |
| | 40°C-4d | Colorless, slightly opalescent, a small number of white particles | NT | NT |
| | 40°C-7d | Colorless, clear, a large number of white particles | 5.9 | 18.0 |
| 10 mM acetic acid-sodium acetate pH 4.6 | Time 0 | Colorless, clear, a small number of white particles | 2.6 | 41.9 |
| | 60°C-3h | Colorless, clear, no visible particles | 3.6 | Multiple peaks |
| | 40°C-4d | Colorless, clear, no visible particles | NT | NT |
| | 40°C-7d | Colorless, clear, a large amount of white fiber | 3.9 | 17.7 |
| 10 mM acetic acid-sodium acetate pH 5.0 | Time 0 | Colorless, slightly opalescent, a large number of white particles | 5.1 | 25.3 |
| | 60°C-3h | Colorless, slightly opalescent, no visible particles | 8.0 | 30.5 |
| | 40°C-4d | Colorless, slightly opalescent, a small amount of visible fiber | NT | NT |
| | 40°C-7d | Colorless, slightly opalescent, a large number of white particles | 5.1 | 24.9 |

| | | | | |
|---|---|---|---|---|
| (Note: h represents hour(s), d represents day(s), particle size reflects colloidal stability; %PD represents dispersion coefficient; NT represents not tested; Imcompleted represents instrumental detection failure due to the poor sample) | | | | |

**Table 27. The results 2 of the anti-ANGPTL3 antibody formulation buffer system confirmation**

| Buffer system | Investigation conditions | SEC | | iCIEF | | | Binding activity (%) |
|---|---|---|---|---|---|---|---|
| | | Aggregate% | Monomer% | Acidic peak% | Main peak% | Basic peak% | |
| 10 mM histidine-histidine hydrochloride pH 6.0 | Time 0 | 5.0 | 95.0 | 16.7 | 71.8 | 11.5 | 109 |
| | 60°C-3h | 28.5 | 71.5 | 16.6 | 50.1 | 33.3 | 75 |
| | 40°C-4d | 5.3 | 94.7 | 17.6 | 69.7 | 12.7 | NT |
| | 40°C-7d | 5.3 | 94.7 | 18.7 | 68.9 | 12.5 | 81 |
| 10 mM succinic acid-sodium succinate pH 4.6 | Time 0 | 6.9 | 93.1 | 15.0 | 71.5 | 13.5 | 107 |
| | 60°C-3h | 93.8 | 6.1 | 6.8 | 28.2 | 65.0 | 65 |
| | 40°C-4d | 18.0 | 82.0 | 17.0 | 64.4 | 18.7 | NT |
| | 40°C-7d | 22.6 | 77.3 | 18.2 | 59.6 | 22.2 | 80 |
| 10 mM acetic acid-sodium acetate pH 4.6 | Time 0 | 7.3 | 92.7 | 15.8 | 71.4 | 12.8 | 106 |
| | 60°C-3h | 88.3 | 11.7 | 8.6 | 34.9 | 56.5 | 77 |
| | 40°C-4d | 9.0 | 90.9 | 16.6 | 68.2 | 15.2 | NT |
| | 40°C-7d | 10.2 | 89.7 | 18.0 | 64.8 | 17.2 | 90 |
| 10 mM acetic acid-sodium acetate pH 5.0 | Time 0 | 5.1 | 94.9 | 16.2 | 72.3 | 11.5 | 110 |
| | 60°C-3h | 89.2 | 10.8 | 10.0 | 36.7 | 53.3 | 67 |
| | 40°C-4d | 5.8 | 94.2 | 17.0 | 69.7 | 13.2 | NT |
| | 40°C-7d | 6.2 | 93.8 | 18.3 | 68.1 | 13.6 | 85 |

| | | | | | | | |
|---|---|---|---|---|---|---|---|
| (Note: h represents hour(s); d represents day(s); NT represents not tested) | | | | | | | |

The results show that: the anti-ANGPTL3 antibody has better stability in the 10 mM histidine-histidine hydrochloride pH 6.0 and 10 mM acetic acid-sodium acetate pH 5.0 buffer systems than in the 10 mM succinic acid-sodium succinate pH 4.6 and 10 mM acetic acid-sodium acetate pH 4.6 buffer systems; where the 10 mM histidine-histidine hydrochloride pH 6.0 buffer system was used, the formulation was opalescent, and after polysorbate 80 was added, the appearance was not improved.

### Example 10: Screening for Sugar Concentrations for Anti-ANGPTL3 Antibody Formulations

The 10 mM acetic acid-sodium acetate pH 5.0 buffer system was selected to prepare buffer systems or anti-ANGPTL3 antibody formulations with different sugar concentrations:
1) 10 mM acetic acid-sodium acetate, pH 5.0;
2) 10 mM acetic acid-sodium acetate, 0.2 mg/mL polysorbate 80, 60 mg/mL sucrose, pH 5.0;
3) 10 mM acetic acid-sodium acetate, 0.2 mg/mL polysorbate 80, 70 mg/mL sucrose, pH 5.0;
4) 10 mM acetic acid-sodium acetate, 0.2 mg/mL polysorbate 80, 75 mg/mL sucrose, pH 5.0;
5) 10 mM acetic acid-sodium acetate, 0.2 mg/mL polysorbate 80, 80 mg/mL sucrose, pH 5.0;
6) 10 mM acetic acid-sodium acetate, 0.2 mg/mL polysorbate 80, 90 mg/mL sucrose, pH 5.0; and
7) 10 mM acetic acid-sodium acetate, 0.2 mg/mL polysorbate 80, 80 mg/mL sucrose, pH 5.0.

The above 1) to 7) all contained 100 mg/mL anti-ANGPTL3 antibody.

The osmotic pressure results for these samples are shown in the table below:

**Table 28. The osmotic pressure results for different sucrose concentrations for the anti-ANGPTL3 antibody**

| No. | Sucrose concentration | Protein concentration | polysorbate 80 concentration | Osmotic pressure (mOsm/kg) |
|---|---|---|---|---|
| 1) | 0mg/mL | 100mg/mL | 0mg/mL | 28 |
| 2) | 60mg/mL | 0mg/mL | 0.2mg/mL | 217 |
| 3) | 70mg/mL | 0mg/mL | 0.2mg/mL | 239 |
| 4) | 75mg/mL | 0mg/mL | 0.2mg/mL | 257 |
| 5) | 80mg/mL | 0mg/mL | 0.2mg/mL | 270 |
| 6) | 90mg/mL | 0mg/mL | 0.2mg/mL | 322 |
| 7) | 80mg/mL | 100mg/mL | 0.2mg/mL | 317 |

According to the osmotic pressure results, the usable concentration of sucrose is 60-90 mg/mL; for example, the sucrose concentration of 75 mg/mL is used to prepare isotonic anti-ANGPTL3 antibody formulations.

### Example 11: Screening for Surfactant Concentrations for Anti-ANGPTL3 Antibody Formulations

10 mM acetic acid-sodium acetate was selected to prepare formulations containing 100 mg/mL anti-ANGPTL3 antibody, 75 mg/mL sucrose and different concentrations of polysorbate 80, and the stability of the antibody under shaking, freeze-thaw, lighting and high-temperature conditions was investigated.
1) 10 mM acetic acid-sodium acetate, 0.1 mg/mL polysorbate 80, 75 mg/mL sucrose, 100 mg/mL anti-ANGPTL3 antibody, pH 5.0
2) 10 mM acetic acid-sodium acetate, 0.4 mg/mL polysorbate 80, 75 mg/mL sucrose, 100 mg/mL anti-ANGPTL3 antibody, pH 5.0

**Table 29. The stability results 1 for the formulations with different surfactant concentrations**

| Formula composition | Standing time | Appearance | Particl e size (nm) | %P D |
|---|---|---|---|---|
| 10 mM acetic acid-sodium acetate +0.1 mg/mL polysorbate 80 +75 mg/mL sucrose +100 mg/mL protein, pH 5.0 | Time 0 | Colorless, slightly opalescent, no visible protein particles | 4.4 | 27.6 |
| | Shaking for 48 h 200 rpm, RT | Colorless, slightly opalescent, no visible protein particles | 4.4 | 25.5 |
| | 3 freeze-thaw cycles -35 °C/RT | Colorless, slightly opalescent, no visible protein particles | 4.8 | 20.0 |
| | 5 freeze-thaw cycles -35 °C/RT | Colorless, slightly opalescent, no visible protein particles | 4.5 | 55.0 |
| | Lighting for 5 d 5000 Lx, 25 °C | Colorless, slightly opalescent, no visible protein particles | 5.3 | 23.7 |
| | Lighting for 10 d 5000 Lx, 25 °C | Colorless, slightly opalescent, no visible protein particles | 4.4 | 27.3 |
| | 40°C-1W | Colorless, slightly opalescent, no visible protein particles | 4.5 | 26.7 |
| | 40°C-2W | Colorless, slightly opalescent, no visible protein particles | 4.6 | 39.7 |
| | 40°C-4W | Colorless, slightly opalescent, no visible protein particles | 4.9 | Mult iple peak s |
| 10 mM acetic acid-sodium acetate +0.4 mg/mL polysorbate 80 +75 mg/mL | Time 0 | Colorless, slightly opalescent, no visible protein particles | 4.3 | 23.9 |
| | Shaking for 48 h 200 rpm, RT | Colorless, slightly opalescent, no visible protein particles | 5.7 | 52.8 |
| sucrose +100 mg/mL protein, pH 5.0 | 3 freeze-thaw cycles -35 °C/RT | Colorless, slightly opalescent, no visible protein particles | 5.1 | 44.8 |
| | 5 freeze-thaw cycles -35 °C/RT | Colorless, slightly opalescent, no visible protein particles | 4.3 | 23.8 |
| | Lighting for 5 d 5000 Lx, 25 °C | Colorless, slightly opalescent, no visible protein particles | 4.4 | 29.3 |
| | Lighting for 10 d 5000 Lx, 25 °C | Colorless, slightly opalescent, no visible protein particles | 4.4 | 24.5 |
| | 40°C-1W | Colorless, slightly opalescent, no visible protein particles | 4.6 | 34.5 |
| | 40°C-2W | Colorless, slightly opalescent, no visible protein particles | 4.4 | 25.4 |
| | 40°C-4W | Colorless, slightly opalescent, no visible protein particles | 4.7 | 34.2 |

| | | | | |
|---|---|---|---|---|
| (Note: h represents hour(s); W represents week(s); RT represents room temperature; particle size reflects colloidal stability; %PD represents dispersion coefficient; NT represents not tested) | | | | |

**Table 30. The stability results 2 for the formulations with different surfactant concentrations**

| Formula composition | Standing time | SEC | | iCIEF | | |
|---|---|---|---|---|---|---|
| | | Aggregate% | Monomer% | Acidic peak% | Main peak% | Basic peak% |
| 10 mM acetic acid-sodium acetate +0.1 mg/mL polysorbate 80 +75 mg/mL sucrose +100 mg/mL protein, pH 5.0 | Time 0 | 2.8 | 97.2 | 16.9 | 78.1 | 5.0 |
| | Shaking for 48 h 200 rpm, RT | 2.9 | 97.1 | 15.1 | 79.3 | 5.5 |
| | 3 freeze-thaw cycles -35 °C/RT | 2.8 | 97.2 | 15.5 | 78.8 | 5.7 |
| | 5 freeze-thaw cycles -35 °C/RT | 2.8 | 97.2 | 15.3 | 78.8 | 5.8 |
| | Lighting for 5 d 5000 Lx, 25 °C | 3.2 | 96.8 | 17.1 | 77.3 | 5.6 |
| | Lighting for 10 d 5000 Lx, 25 °C | 3.3 | 96.7 | 17.1 | 78.0 | 4.9 |
| | 40°C-1W | 3.4 | 96.6 | 20.2 | 72.0 | 7.8 |
| | 40°C-2W | 4.1 | 95.8 | 22.1 | 68.9 | 9.0 |
| | 40°C-4W | 5.9 | 93.9 | 30.4 | 59.5 | 10.1 |
| 10 mM acetic acid-sodium acetate +0.4 mg/mL polysorbate 80 +75 mg/mL sucrose +100 mg/mL protein, pH 5.0 | Time 0 | 2.8 | 97.2 | 16.4 | 78.8 | 4.8 |
| | Shaking for 48 h 200 rpm, RT | 2.8 | 97.2 | 15.9 | 77.3 | 6.8 |
| | 3 freeze-thaw cycles -35 °C/RT | 2.8 | 97.2 | 15.3 | 78.9 | 5.8 |
| | 5 freeze-thaw cycles -35 °C/RT | 2.8 | 97.2 | 16.1 | 78.4 | 5.5 |
| | Lighting for 5 d 5000 Lx, 25 °C | 3.1 | 96.9 | 17.1 | 76.7 | 6.2 |
| | Lighting for 10 d 5000 Lx, 25 °C | 3.3 | 96.6 | 18.0 | 76.6 | 5.4 |
| | 40°C-1W | 3.4 | 96.6 | 19.9 | 73.4 | 6.7 |
| | 40°C-2W | 4.2 | 95.7 | 21.6 | 70.7 | 7.7 |
| | 40°C-4W | 6.0 | 93.8 | 30.2 | 59.8 | 10.1 |

| | | | | | | |
|---|---|---|---|---|---|---|
| (Note: h represents hour(s); W represents week(s); RT represents room temperature) | | | | | | |

The results show that: in the anti-ANGPTL3 antibody formulation containing 0.1 mg/mL or 0.4 mg/mL polysorbate 80, there was no significant difference in stability under shaking, freeze-thaw, lighting and high-temperature conditions. Thus, the usable concentration range for polysorbate 80 in anti-ANGPTL3 antibody formulations is 0.1 mg/mL-0.4 mg/mL.

### Example 12: Effect of Formulation's pH on Stability

10 mM acetic acid-sodium acetate was selected to prepare formulations containing 75 mg/mL sucrose, 0.2 mg/mL polysorbate 80 and 100 mg/mL anti-ANGPTL3 antibody, with the pH values of 5.0 and 5.4, respectively, and the stability of the antibody formulations under shaking, freeze-thaw, lighting and high-temperature conditions was investigated.
1) 10 mM acetic acid-sodium acetate, 0.2 mg/mL polysorbate 80, 75 mg/mL sucrose, 100 mg/mL anti-ANGPTL3 antibody, pH 5.0
2) 10 mM acetic acid-sodium acetate, 0.2 mg/mL polysorbate 80, 75 mg/mL sucrose, 100 mg/mL anti-ANGPTL3 antibody, pH 5.4

**Table 31. The results 1 of the investigation of the effect of the formulation's pH on stability**

| Formula composition | Standing time | Appearance | Particle size (nm) | %PD |
|---|---|---|---|---|
| 10 mM acetic acid-sodium acetate +0.2 mg/mL polysorbate 80 +75 mg/mL sucrose +100 mg/mL protein, pH 5.0 | Time 0 | Colorless, slightly opalescent, no visible protein particles | 5.4 | Multiple peaks |
| | Shaking for 48 h 200 rpm, RT | Colorless, slightly opalescent, no visible protein particles | 4.8 | Multiple peaks |
| | 3 freeze-thaw cycles -35 °C/RT | Colorless, slightly opalescent, no visible protein particles | 5.0 | Multiple peaks |
| | 5 freeze-thaw cycles -35 °C/RT | Colorless, slightly opalescent, no visible protein particles | 5.0 | Multiple peaks |
| | Lighting for 5 d 5000 Lx, 25 °C | Colorless, slightly opalescent, no visible protein particles | 7.1 | Multiple peaks |
| | Lighting for 10 d 5000 Lx, 25 °C | Colorless, slightly opalescent, no visible protein particles | 5.4 | Multiple peaks |
| | 40°C-1W | Colorless, slightly opalescent, no visible protein particles | 6.3 | Multiple peaks |
| | 40°C-2W | Colorless, slightly opalescent, no visible protein particles | 6.3 | Multiple peaks |
| | 40°C-4W | Colorless, slightly opalescent, no visible protein particles | 5.6 | Multiple peaks |
| 10 mM acetic acid-sodium acetate +0.2 mg/mL polysorbate 80 +75 mg/mL sucrose +100 mg/mL protein, pH 5.4 | Time 0 | Colorless, slightly opalescent, no visible protein particles | 6.1 | 29.6 |
| | Shaking for 48 h 200 rpm, RT | Colorless, slightly opalescent, no visible protein particles | 6.4 | 36.6 |
| | 3 freeze-thaw cycles -35 °C/RT | Colorless, slightly opalescent, no visible protein particles | 6.5 | 36.3 |
| | 5 freeze-thaw cycles -35 °C/RT | Colorless, slightly opalescent, no visible protein particles | 8.1 | Multiple peaks |
| | Lighting for 5 d 5000 Lx, 25 °C | Colorless, slightly opalescent, no visible protein particles | 8.1 | Multiple peaks |
| | Lighting for 10 d 5000 Lx, 25 °C | Colorless, slightly opalescent, no small number of visible protein particles | 6.3 | 30.5 |
| | 40°C-1W | Colorless, slightly opalescent, no visible protein particles | 6.4 | 34.3 |
| | 40°C-2W | Colorless, slightly opalescent, no visible protein particles | 6.6 | 38.3 |
| | 40°C-4W | Colorless, slightly opalescent, no visible protein particles | 6.9 | 48.7 |

| | | | | |
|---|---|---|---|---|
| (Note: h represents hour(s); W represents week(s); RT represents room temperature; particle size reflects colloidal stability; %PD represents dispersion coefficient) | | | | |

**Table 32. The results 2 of the investigation of the effect of the formulation's pH on stability**

| Formula composition | Standing time | SEC | | iCIEF | | |
|---|---|---|---|---|---|---|
| | | Aggregate% | Monomer% | Acidic peak% | Main peak% | Basic peak% |
| 10 mM acetic acid-sodium acetate +0.2 mg/mL polysorbate 80 +75 mg/mL sucrose +100 mg/mL protein, pH 5.0 | Time 0 | 3.6 | 96.4 | 18.9 | 74.0 | 7.1 |
| | Shaking for 48 h 200 rpm, RT | 3.6 | 96.3 | 18.2 | 75.2 | 6.6 |
| | 3 freeze-thaw cycles -35 °C/RT | 3.6 | 96.4 | 18.1 | 75.2 | 6.7 |
| | 5 freeze-thaw cycles -35 °C/RT | 3.6 | 96.4 | 17.6 | 76.1 | 6.3 |
| | Lighting for 5 d 5000 Lx, 25 °C | 4.0 | 95.9 | 18.4 | 73.3 | 8.4 |
| | Lighting for 10 d 5000 Lx, 25 °C | 4.3 | 95.6 | 19.2 | 73.1 | 7.8 |
| | 40°C-1W | 5.1 | 94.9 | 21.2 | 69.7 | 9.1 |
| | 40°C-2W | 6.3 | 93.6 | 25.3 | 64.7 | 10.0 |
| | 40°C-4W | 10.4 | 89.4 | 38.4 | 50.8 | 10.7 |
| 10 mM acetic acid-sodium acetate +0.2 mg/mL polysorbate 80 +75 mg/mL sucrose +100 mg/mL | Time 0 | 3.1 | 96.9 | 16.8 | 78.3 | 4.9 |
| | Shaking for 48 h 200 rpm, RT | 3.2 | 96.8 | 15.5 | 79.5 | 5.0 |
| | 3 freeze-thaw cycles -35 °C/RT | 3.2 | 96.8 | 15.3 | 78.8 | 5.9 |
| | 5 freeze-thaw cycles -35 °C/RT | 3.2 | 96.8 | 16.3 | 77.3 | 6.4 |
| protein, pH 5.4 | Lighting for 5 d 5000 Lx, 25 °C | 3.6 | 96.4 | 18.1 | 76.0 | 5.9 |
| | Lighting for 10 d 5000 Lx, 25 °C | 3.8 | 96.2 | 19.0 | 74.7 | 6.3 |
| | 40°C-1W | 3.7 | 96.2 | 20.0 | 73.7 | 6.4 |
| | 40°C-2W | 4.3 | 95.7 | 23.4 | 67.3 | 9.3 |
| | 40°C-4W | 5.3 | 94.5 | 31.2 | 59.3 | 9.5 |

| | | | | | | |
|---|---|---|---|---|---|---|
| (Note: h represents hour(s); W represents week(s); RT represents room temperature) | | | | | | |

The results show that: compared to time 0, the pH 5.0 and pH 5.4 formulations showed similar trends of change in protein under shaking, freeze-thaw, lighting and high-temperature conditions. Thus, the pH range for anti-ANGPTL3 antibody formulations was determined to be 5.0-5.4.

0.1 mg/mL polysorbate 80 and 0.4 mg/mL polysorbate 80 showed little influence on the stability of the antibody, so the usable concentration range for polysorbate 80 in formulations is 0.1-0.4 mg/mL; the pH value of 5.0 and 5.4 showed little influence on the stability of the antibody, so the usable pH range for formulations is 5.0-5.4; the concentration of the anti-ANGPTL3 antibody was determined at 100 mg/mL.

### Example 13: Alternative Formulas

In addition, the present disclosure further provides pharmaceutical formulations of the anti-ANGPTL3 antibody of other formulas, including but not limited to:
(1) 100 mg/mL anti-ANGPTL3 antibody, 75 mg/mL sucrose, 0.1 mg/mL polysorbate 80, and 10 mM acetic acid-sodium acetate buffer pH 5.0;
(2) 100 mg/mL anti-ANGPTL3 antibody, 75 mg/mL sucrose, 0.2 mg/mL polysorbate 80, and 10 mM acetic acid-sodium acetate buffer pH 5.0;
(3) 100 mg/mL anti-ANGPTL3 antibody, 75 mg/mL sucrose, 0.4 mg/mL polysorbate 80, and 10 mM acetic acid-sodium acetate buffer pH 5.0;
(4) 100 mg/mL anti-ANGPTL3 antibody, 75 mg/mL sucrose, 0.1 mg/mL polysorbate 80, and 10 mM acetic acid-sodium acetate buffer pH 5.2;
(5) 100 mg/mL anti-ANGPTL3 antibody, 75 mg/mL sucrose, 0.2 mg/mL polysorbate 80, and 10 mM acetic acid-sodium acetate buffer pH 5.2;
(6) 100 mg/mL anti-ANGPTL3 antibody, 75 mg/mL sucrose, 0.4 mg/mL polysorbate 80, and 10 mM acetic acid-sodium acetate buffer pH 5.2;
(7) 100 mg/mL anti-ANGPTL3 antibody, 75 mg/mL sucrose, 0.1 mg/mL polysorbate 80, and 10 mM acetic acid-sodium acetate buffer pH 5.4;
(8) 100 mg/mL anti-ANGPTL3 antibody, 75 mg/mL sucrose, 0.2 mg/mL polysorbate 80, and 10 mM acetic acid-sodium acetate buffer pH 5.4;
(9) 100 mg/mL anti-ANGPTL3 antibody, 75 mg/mL sucrose, 0.4 mg/mL polysorbate 80, and 10 mM acetic acid-sodium acetate buffer pH 5.4;
(10) 80 mg/mL, 100 mg/mL, 120 mg/mL or 150 mg/mL anti-ANGPTL3 antibody, 75 mg/mL sucrose, 0.2 mg/mL polysorbate 80, and 10 mM acetic acid-sodium acetate buffer, pH 5.0 to 5.4; and
(11) 100 mg/mL anti-ANGPTL3 antibody, 60 mg/mL, 70 mg/mL, 75 mg/mL, 80 mg/mL, 85 mg/mL or 90 mg/mL sucrose, 0.2 mg/mL polysorbate 80, and 10 mM acetic acid-sodium acetate buffer, pH 5.0 to 5.4.

The experimental results show that anti-ANGPTL3 antibody formulations of the above formulas have good stability; the above formulas can be applied to the preparation of anti-ANGPTL3 antibody drugs.

## Claims

1. A pharmaceutical composition, comprising an anti-ANGPTL3 antibody or an antigen-binding fragment thereof, and a buffer, wherein the buffer is selected from any of acetic acid-sodium acetate, succinic acid-sodium succinate, histidine-hydrochloride, and citric acid-sodium citrate buffers, and is preferably an acetic acid-sodium acetate or succinic acid-sodium succinate buffer; wherein the anti-ANGPTL3 antibody or the antigen-binding fragment thereof comprises an antibody heavy chain variable region and an antibody light chain variable region, wherein the heavy chain variable region comprises a HCDR1, a HCDR2 and a HCDR3, the amino acid sequences of which are set forth in SEQ ID NO: 14, SEQ ID NO: 15 and SEQ ID NO: 16, respectively; and the light chain variable region comprises a LCDR1, a LCDR2 and a LCDR3, the amino acid sequences of which are set forth in SEQ ID NO: 11, SEQ ID NO: 12 and SEQ ID NO: 13, respectively.

2. The pharmaceutical composition according to claim 1, wherein the buffer has a pH of 4.0 to 7.0, preferably 5.0 to 5.4, and most preferably about 5.2.

3. The pharmaceutical composition according to claim 1 or 2, wherein the concentration of the buffer is 5 mM to 40 mM, preferably 5 mM to 20 mM, and most preferably about 10 mM.

4. The pharmaceutical composition according to any of claims 1 to 3, wherein the concentration of the anti-ANGPTL3 antibody or the antigen-binding fragment thereof is 10 mg/mL to 200 mg/mL, preferably 80 mg/mL to 120 mg, and most preferably about 100 mg/mL.

5. The pharmaceutical composition according to any of claims 1 to 4, further comprising a surfactant, wherein the surfactant is preferably polysorbate 80 or polysorbate 20, more preferably polysorbate 80.

6. The pharmaceutical composition according to claim 5, wherein the concentration of the surfactant is 0.05 mg/mL to 0.6 mg/mL, preferably 0.1 mg/mL to 0.4 mg/mL, and more preferably about 0.2 mg/mL.

7. The pharmaceutical composition according to any of claims 1 to 6, further comprising a stabilizer, wherein the stabilizer is a sugar; preferably, the sugar is selected from the group consisting of sucrose, trehalose, mannitol and sorbitol; more preferably, the sugar is sucrose.

8. The pharmaceutical composition according to claim 7, wherein the concentration of the sugar is 60 mg/mL to 90 mg/mL, preferably 70 mg/mL to 80 mg/mL, and most preferably about 75 mg/mL.

9. The pharmaceutical composition according to any of claims 1 to 8, comprising:
(a) 10 mg/mL to 200 mg/mL anti-ANGPTL3 antibody or antigen-binding fragment thereof,
(b) 5 mM to 30 mM acetate buffer,
(c) 60 mg/mL to 90 mg/mL sucrose, and
(d) 0.05 mg/mL to 0.6 mg/mL polysorbate 80,
wherein the pharmaceutical composition preferably has a pH of 4.5 to 6.5, and more preferably has a pH of 5.0 to 5.4.

10. The pharmaceutical composition according to any of claims 1 to 9, comprising:
(a) about 100 mg/mL anti-ANGPTL3 antibody or antigen-binding fragment thereof,
(b) 5 mM to 15 mM acetic acid-sodium acetate buffer,
(c) 60 mg/mL to 90 mg/mL sucrose, and
(d) 0.1 mg/mL to 0.4 mg/mL polysorbate 80,
wherein the pharmaceutical composition preferably has a pH of 5.0 to 5.4, and more preferably has a pH of about 5.2.

11. The pharmaceutical composition according to any of claims 1 to 10, wherein the anti-ANGPTL3 antibody or the antigen-binding fragment thereof is a murine, chimeric or humanized antibody; preferably, the light and heavy chain FR sequences of the humanized antibody are derived from human germline light and heavy chain FRs, respectively.

12. The pharmaceutical composition according to any of claims 1 to 11, wherein the amino acid sequence of the heavy chain variable region is set forth in SEQ ID NO: 17 or has at least 90% sequence identity thereto, and the amino acid sequence of the light chain variable region is set forth in SEQ ID NO: 18 or has at least 90% sequence identity thereto.

13. The pharmaceutical composition according to any of claims 1 to 12, wherein the anti-ANGPTL3 antibody or the antigen-binding fragment thereof comprises an antibody heavy chain and an antibody light chain, wherein: the amino acid sequence of the heavy chain is set forth in SEQ ID NO: 21 or has at least 90% sequence identity thereto, and the amino acid sequence of the light chain is set forth in SEQ ID NO: 22 or has at least 90% sequence identity thereto.

14. A method for preparing the pharmaceutical composition according to any of claims 1 to 13, comprising the step of subjecting a stock solution of the anti-ANGPTL3 antibody or the antigen-binding fragment thereof to a buffer exchange, wherein the buffer is preferably an acetic acid-sodium acetate buffer.

15. A lyophilized formulation comprising an anti-ANGPTL3 antibody or an antigen-binding fragment thereof, wherein the lyophilized formulation is obtained by lyophilizing the pharmaceutical composition according to any of claims 1 to 13; preferably, the lyophilizing comprises the steps of pre-freezing, primary drying and secondary drying in sequence.

16. A reconstituted solution comprising an anti-ANGPTL3 antibody or an antigen-binding fragment thereof, wherein the reconstituted solution is obtained by reconstituting the lyophilized formulation according to claim 15.

17. An article of manufacture, comprising a container, wherein the container contains the pharmaceutical composition according to any of claims 1 to 13, or the lyophilized formulation according to claim 15, or the reconstituted solution according to claim 16.

18. Use of the pharmaceutical composition according to any of claims 1 to 13, or the lyophilized formulation according to claim 15, or the reconstituted solution according to claim 16, in the preparation of a medicament for treating and/or preventing a disease or disorder;
wherein preferably, the disease or disorder is selected from one or more of hyperlipidemia, hyperlipoproteinemia, dyslipidemia, cardiovascular diseases or disorders, cancer or tumors, non-neoplastic angiogenesis-related diseases or disorders, and inflammatory diseases or disorders;
more preferably, the disease or disorder is selected from one or more of atherosclerotic dyslipidemia, diabetic dyslipidemia, hypertriglyceridemia, hypercholesterolemia, chylomicronemia, mixed dyslipidemia, lipodystrophy, lipoatrophy, atherosclerosis, aneurysm, hypertension, angina pectoris, strokes, cerebrovascular diseases, congestive heart failure, coronary artery disease, myocardial infarction, peripheral vascular disease, acute pancreatitis, nonalcoholic steatohepatitis (NASH), blood glucose abnormality, age-related macular degeneration, central or branch retinal vein occlusion, diabetic retinopathy, retinopathy of prematurity, arthritis, rheumatoid arthritis (RA), and psoriasis.

19. A method for treating and/or preventing a disease or disorder, comprising administering to a subject in need thereof the pharmaceutical composition according to any of claims 1 to 13, or the reconstituted solution according to claim 16;
wherein preferably, the disease or disorder is selected from one or more of hyperlipidemia, hyperlipoproteinemia, dyslipidemia, cardiovascular diseases or disorders, cancer or tumors, non-neoplastic angiogenesis-related diseases or disorders, and inflammatory diseases or disorders;
more preferably, the disease or disorder is selected from one or more of atherosclerotic dyslipidemia, diabetic dyslipidemia, hypertriglyceridemia, hypercholesterolemia, chylomicronemia, mixed dyslipidemia, lipodystrophy, lipoatrophy, atherosclerosis, aneurysm, hypertension, angina pectoris, strokes, cerebrovascular diseases, congestive heart failure, coronary artery disease, myocardial infarction, peripheral vascular disease, acute pancreatitis, nonalcoholic steatohepatitis (NASH), blood glucose abnormality, age-related macular degeneration, central or branch retinal vein occlusion, diabetic retinopathy, retinopathy of prematurity, arthritis, rheumatoid arthritis (RA), and psoriasis.
